# EUROPEAN PATENT SPECIFICATION

(11) **EP 1 909 897 B1**
(45) Date of publication and mention of the grant of the patent: **10.05.2017**
(21) Application number: 06786721.8
(22) Date of filing: 07.07.2006
(51) Int. Cl.: A61B 5/0245, A61B 5/00, A61B 5/0215, A61B 5/029, A61B 5/08, A61B 5/145, A61B 5/11, A61N 1/365, A61N 1/37

(54) **MONITORING PHYSIOLOGICAL RESPONSES TO STEADY STATE ACTIVITY**
ÜBERWACHUNG DER PHYSIOLOGISCHEN REAKTIONEN AUF STEADY-STATE-AKTIVITÄT
CONTROLE DE REPONSES PHYSIOLOGIQUES A UNE ACTIVITE STATIONNAIRE

(30) Priority: 19.07.2005 US 184327
(43) Date of publication of application: 16.04.2008
(60) Divisional application: 10189876.5
(73) Proprietor: Cardiac Pacemakers, Inc., St. Paul MN 55112-5798 (US)
(72) Inventor: BECK, Kenneth, St. Paul, Minnesota 55104 (US); BAKER, Lemont, Raleigh, North Carolina 27613 (US); HOPPER, Donald, Maple Grove, Minnesota 55369 (US)
(74) Representative: Pfenning, Meinig & Partner mbB
(86) International application number: PCT/US2006/026660
(87) International publication number: WO 2007/011565

(56) References cited:
- EP-A2- 0 702 980
- WO-A-01/23040
- WO-A2-2005/037077
- US-A- 5 931 858
- US-A1- 2003 149 453
- US-A1- 2003 204 147
- US-A1- 2005 065 443
- US-B1- 6 904 313

## Description

### TECHNICAL FIELD

This patent document pertains generally to measuring physiological activity, and more particularly, but not by way of limitation, to methods and apparatus for monitoring physiological responses to steady state activity.

### BACKGROUND

Implantable devices such as pacers and defibrillators detect and process physiological data. For example, some devices detect an intrinsic electrical heart signal using one or more electrodes on a lead that is coupled to the device. Pacers, for example, use sensed data to adjust the target heart rate of a patient. Defibrillators analyze heart activity to assess whether an antitachyarrhythmia therapy is needed. Other parameters such as blood pressure are also detectable using implantable sensors.

### SUMMARY

The method of the invention includes detecting a physical activity signal, using an implantable medical device, from a human or animal subject, detecting a different other physiological signal, from the subject, using the implantable medical device. The method further includes processing the physical activity signal to define a first time period associated with a first steady-state physical activity level of the subject, including identifying for the first time period a first beginning time at which the physical activity of the subject is deemed to be at steady state at the first steady-state physical activity level, and a first ending time at which the subject leaves the first steady-state physical activity level. The method further includes processing the physical activity signal to define a second time period associated with a second steady-state physical activity level of the subject, including identifying for the second time period a second beginning time at which the physical activity of the subject is deemed to be at steady state at the second steady-state physical activity level, and a second ending time at which the subject leaves the second steady-state physical activity level. The method also includes obtaining a first indicator by combining data from the other physiological signal obtained during the first time period, obtaining a second indicator by combining data from the other physiological signal obtained during the second time period, and using the first and second indicators to provide a resulting indicator. The machine-assisted method comprising detecting a physical activity signal, using an implantable medical device, from a human or animal subject, the physical activity signal falling within an activity range divided into activity bins, and contemporaneously detecting a different other physiological signal, from the subject, using the implantable medical device. The method further includes processing the physical activity signal to define a first time period associated with a first steady-state physical activity level of the subject, including identifying for the first time period a first beginning time at which the physical activity of the subject is deemed to be at steady state at the first steady-state physical activity level, and a first ending time at which the subject leaves the first steady-state physical activity level, the first steady-state physical activity level corresponding to an activity bin. The method also includes processing the physical activity signal to define a second time period associated with a second steady-state physical activity level of the subject, the second steady-state physical activity level of the subject corresponding to the same activity bin as the first steady-state physical activity level of the subject, including identifying for the second time period a second beginning time at which the physical activity of the subject is deemed to be at steady state at the second steady-state physical activity level, and a second ending time at which the subject leaves the second steady-state physical activity level. The method further includes combining data from the other physiological signal also obtained during the first and second time periods, and using the combined data from the other physiological signal to provide a resulting indicator.The method includes detecting a plurality of activity levels using an accelerometer, determining for a candidate activity level an activity level bin value corresponding to a range of activity level values, and determining whether the candidate activity level was at steady state using bin values from activity levels detected before and after the candidate activity level. If the candidate activity level was at steady state, physiological data detected contemporaneously with the candidate activity level using an implantable medical device is added to one or more sum registers, and one or more counters is incremented. If the candidate activity level was not at steady state and a previous activity level was at steady state, the physiological data is averaged using the one or more sum registers and one or more counters. The method further includes aggregating the averaged physiological data with previous physiological data detected at an activity level corresponding to the same bin value, and storing aggregated physiological data averages.

### BRIEF DESCRIPTION OF THE DRAWINGS

FIG. 1 is a flow chart that illustrates an example method of monitoring physiological responses to steady state activity and providing a resulting indicator.
FIG. 2 is a flow chart that illustrates an example method of monitoring physiological responses to steady state activity, aggregating physiological data according to periods of steady state activity, and providing a resulting indicator.
FIGS. 3A and 3B are flow charts that illustrate an example method that includes analyzing a candidate activity level to identify steady state activity.
FIGS. 4A and 4B are flow charts that schematically illustrate an example method of monitoring physiological responses to steady state activity using activity bins.
FIGS. 5A and 5B are schematic illustrations of an example implantable device including circuits for detecting a physiological response to steady-state activity.
FIG. 6A is an example plot of heart and activity against time.
FIG. 6B is a table that illustrates example data that can be obtained using methods and apparatus for detecting a physiological response to steady-state activity.
FIG. 7A is an example graph that shows heart rate plotted against activity.
FIG. 7B is an example graph that shows heart rate plotted against activity for two instances in time.
FIG. 8 is an example graph that shows heart rate at 50 mG and heart rate slope (dHR/dActivity) plotted against days from baseline.
FIG. 9 is an example graph that shows stroke volume plotted against activity.
FIG. 10 is an example graph that shows stroke volume plotted against heart rate.
FIG. 11 is an example graph that shows time plotted against activity.
FIG. 12 is an example graph that shows tidal volume plotted against activity.
FIG. 13 is an example graph that shows respiration rate plotted against activity.
FIG. 14 is an example graph that shows respiratory rate plotted against tidal volume.
FIG. 15 is an example graph that shows pulmonary artery diastolic pressure plotted against activity.
FIG. 16 is an example graph that shows stroke volume plotted against pulmonary artery diastolic pressure.
FIG. 17 is an example graph that shows cardiac output plotted against activity.
FIG. 18 is an example graph that shows venous blood oxygen saturation plotted against activity.
FIG. 19 is an example graph that shows minute ventilation plotted against activity.
FIG. 20 is an example graph that shows minute ventilation plotted against cardiac output.

### DETAILED DESCRIPTION

The following detailed description includes references to the accompanying drawings, which form a part of the detailed description. The drawings show, by way of illustration, specific embodiments in which the invention may be practiced. These embodiments are also referred to herein as "examples." The following detailed description is not to be taken in a limiting sense, and the scope of the present invention is defined by the appended claims and their equivalents.

Physiological responses to periods of steady state activity are monitored using physiological sensors and processing circuitry. In an example, an implantable device includes one or more sensors that detect physiological parameters such as heart rate, tidal volume, respiratory rate, and/or total minute ventilation. The implantable device can also detect activity, for example using an accelerometer. In an example, the implantable device also includes a posture sensor. Periods of steady state activity are identified by monitoring activity data (e.g. accelerometer output) and deeming steady state to exist when the activity data meets certain criteria. In an example, a period of steady state activity is associated with a particular activity range, or "bin." Steady state physiological data is stored and associated with a particular bin. In an example, physiological data is aggregated over a period of time (e.g. 24 hours) for multiple instances of activity in one or more activity ranges. In an example, steady state physiological data from different points in time (e.g. 30 days apart) is compared or otherwise processed to determine wellness and/or identify possible physiological conditions or changes.

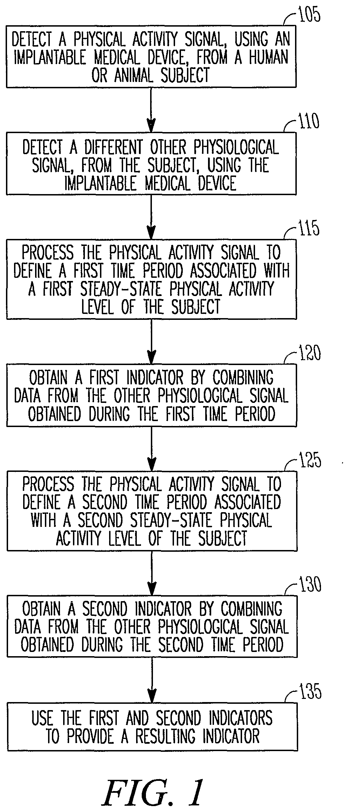

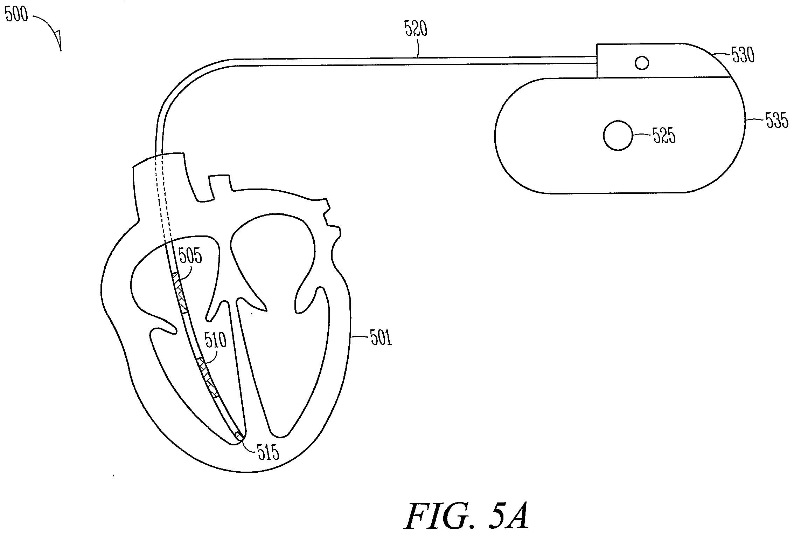

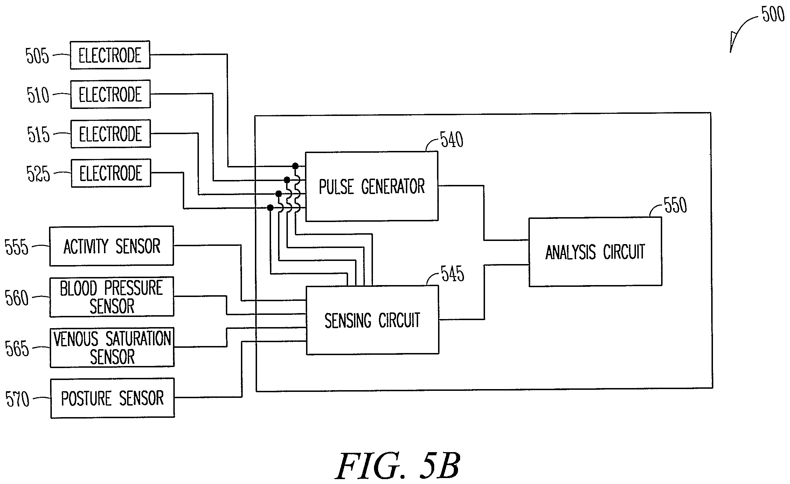

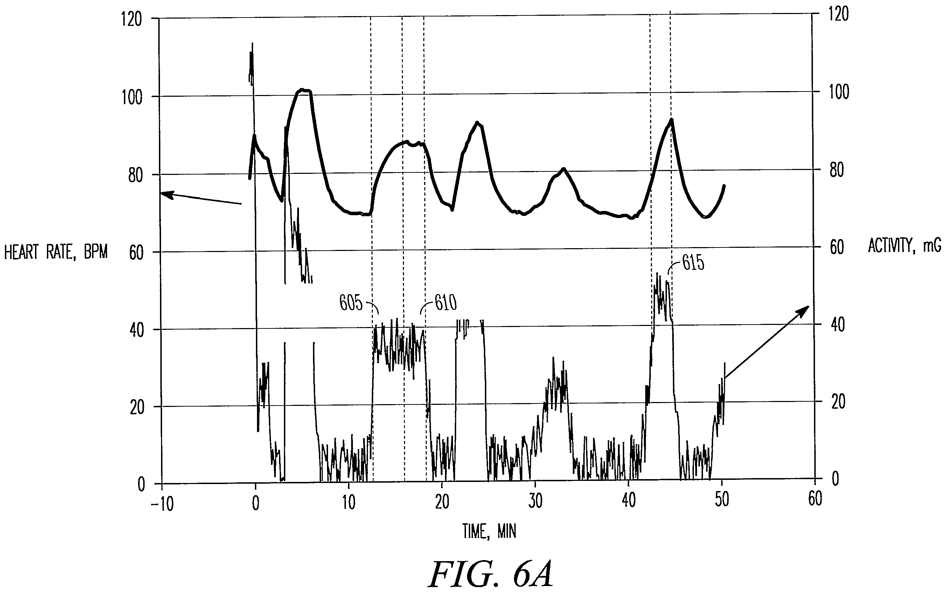

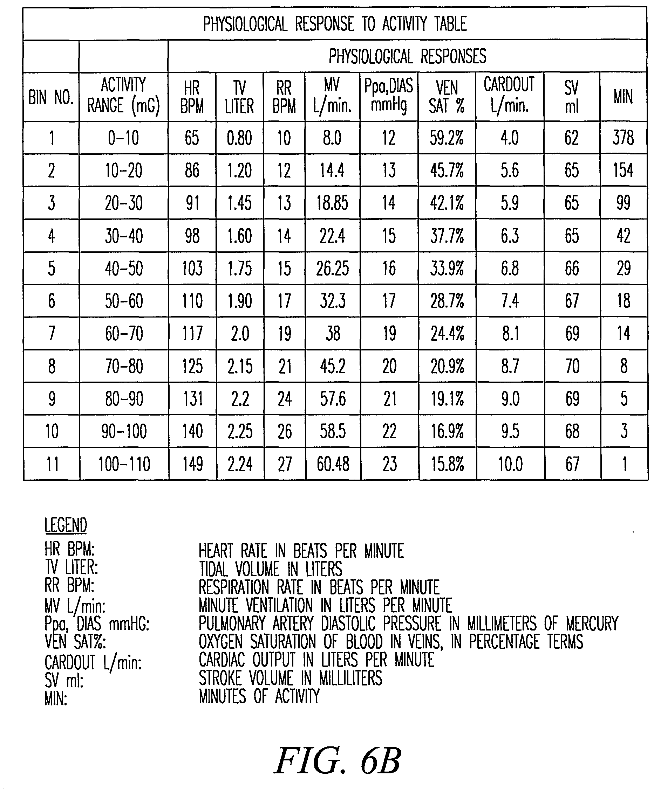

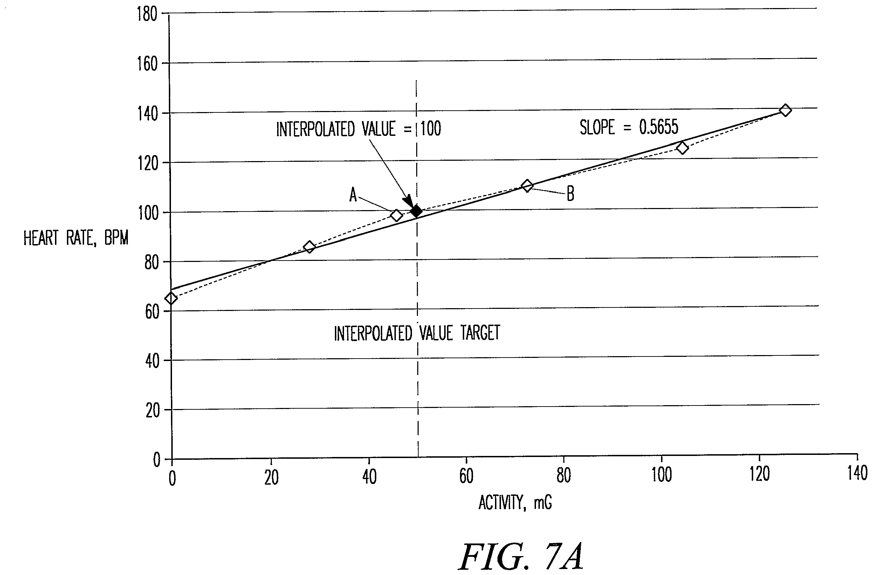

FIG. 1, 2, 3A, 3B, 4A, and 4B are flow chart that illustrate example methods of monitoring physiological responses to steady state activity. FIGS. 5A and 5B are schematic illustrations of an example implantable device including circuits for detecting a physiological response to steady-state activity. FIG. 6A is a graph that shows heart beat and activity plotted against time. FIG. 6B is a table that illustrates example data that can be obtained using methods and apparatus for detecting physiological responses to steady-state activity. FIGS. 7A-20 are graphs that show relationships of detected physiological parameters and indicators that can be obtained from the relationships.

Referring now to the example method schematically illustrated in the flow chart of FIG. 1, at 105, a physical activity signal is detected from a human or animal subject using an implantable medical device. In an example, the physical activity signal is detected using an accelerometer, which is optionally coupled to or contained within a device such as a pacer, defibrillator, neural stimulator, or other device. At 110, a different other physiological signal is detected from the subject using the implantable medical device. In an example, the different other physiological signal is detected using a sensor that senses, for example, an intrinsic electrical heart signal, heart sounds, thoracic impedance, blood oxygen saturation, or intravascular blood pressure. In an example, the different other physiological signal includes heart rate, respiratory rate, stroke volume, tidal volume, venous blood oxygen saturation, cardiac output, or a blood pressure such as pulmonary artery diastolic pressure, systolic pressure, or mean pressure. At 115, the physical activity signal is processed to define a first time period associated with a first steady-state physical activity level of the subject. In an example, the physical activity signal corresponds to a bin associated with a range of activity signal values. In an example, processing the physical activity signal includes identifying for the first time period a first beginning time at which the physical activity of the subject is deemed to be at steady state at the first steady-state physical activity level, and a first ending time at which the subject leaves the first steady-state physical activity level. In an example, the physical activity of the subject is deemed to be at steady state when the detected activity has exhibited certain characteristics that are indicative of steady state for an amount of time. In an example, the physical activity of the subject is deemed to be at steady state when the activity stays within a "bin range" of activity signal values for a specified period of time. In one example, the activity is deemed to be at steady state if the detected activity has been within a defined range (e.g. 10 milligrams (mG)) for a period of 20 seconds. In another example, the activity is deemed to be steady state if the detected activity has been within a defined range (e.g. 20 mG) for a period of two minutes. In an example, two ranges are used - a first, smaller range (e.g. 10 mG) for a smaller period of time (e.g. one accelerometer reading or 20 seconds), and a second, larger range (e.g. 20 mG) for a larger period of time (e.g. two minutes). It is understood that an accelerometer is not necessarily calibrated to mG, and could alternatively be calibrated to a different unit, or be uncalibrated, in which case ranges could be defined in raw detected values. In an example, the physical activity is deemed to have left the steady state when the difference between a particular activity bin value and a previous activity bin value or a sequence of activity bin values exceeds a defined value. In an example, the algorithm for determining whether a detected activity remains in steady state (or has left steady state) is the same as the algorithm for determining whether the activity has entered steady state.

FIG. 6A shows an example of heart rate (in beats per minutes (BPM)) and activity (in milli-G's) plotted against a time axis. The plot shows a lag between steady state detected activity and steady state physiological conditions (i.e. heart rate). In an example, for the purpose of using physiological data to provide a resulting indicator, physical activity is deemed to be at steady state after the detected physical activity has been in a specified range for a specified period of time. In an example, the specified period of time is about two or three minutes. For example, FIG. 6A shows a time period 610 from around minute 16 to minute 18, which is considered steady state physical activity. The graph shows steady state detected activity between about 30 mG and 40 mG during the time period 610. The time period 605 from around minute 13 to minute 16 also shows constant detected activity in this range. In an example, the three minute period 605 from minute 13 to minute 16 qualifies the following period 610 for consideration as steady state activity. This allows time for the heart rate to reach steady state after the detected activity level has reached a steady state. For example, the plot of heart rate against time shows that the heart rate moves to a steady state value of around 88 beats per minute during the period 610 from minute about 16 to minute 18. In contrast, during time period 605, the heart rate is not steady state, but moves from about 70 beats per minute to around 88 beats per minute. During period 615 from about minute 43 to 45, the detected activity is within a range of about 42 to 52 mG, but the period is to short for the heart beat to reach steady state, as can be seen from the graph.

Returning to FIG. 1, at 120, a first indicator is obtained by combining data from the other physiological signal (e.g. tidal volume) obtained during the first period. In an example, the data is combined in an average (mean), a weighted average, a median, a range, or a standard deviation. In an example, obtaining the first indicator includes combining the data from the physiological signal obtained during the first period also with data from the physiological sensor obtained from a previous steady state period that falls into the same bin as the data obtained during the first period.

Referring again to FIG. 1, at 125, the physical activity signal is processed to define a second time period associated with a second steady-state physical activity level of the subject. It is understood that "first" and "second" are used herein to differentiate the time periods and do not refer to a sequential order. For example, the first period could come after the second period, or a third period could occur between the first and second period. In an example, the processing of the physical activity signal includes identifying for the second time period a second beginning time at which the physical activity of the subject is deemed to be at steady state at a second steady-state physical activity level, and a second ending time at which the subject leaves the second steady-state physical activity level. In an example, the second steady-state physical activity level corresponds to a different bin than the first steady-state activity level. In another example, the second steady-state physical activity level corresponds to the same bin as the first steady-state activity level, in which case the data from other physiological signal obtained during the first and second time periods is optionally combined. At 130, a second indicator is obtained by combining data from the other physiological signal obtained during the second time period. In an example, one or more additional other physiological signals are detected from the subject during periods of steady state activity, and one or more additional indicators are determined using the additional physiological indicators. In an example, respiratory rate, pulmonary artery diastolic pressure, and tidal volume are detected concurrently during steady state periods.

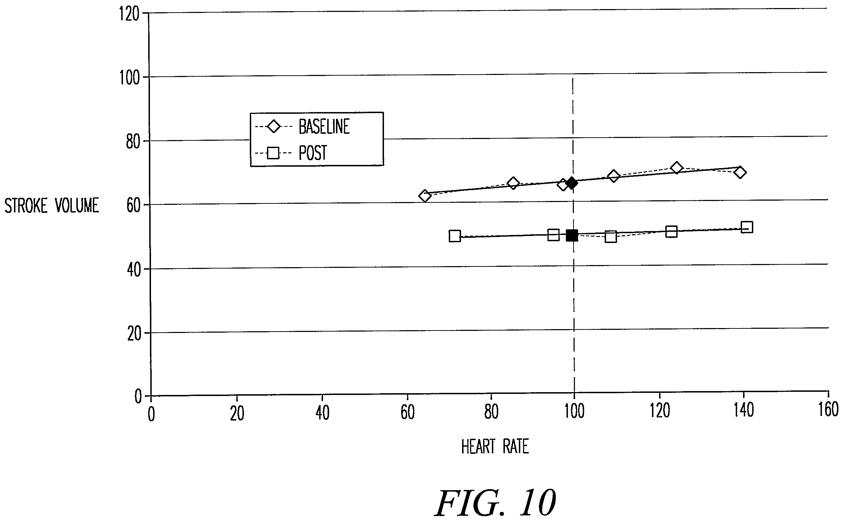

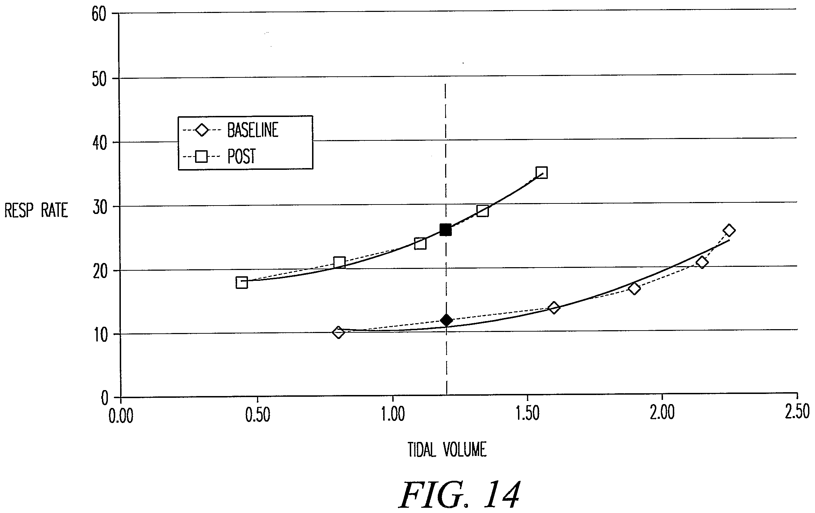

At 135, the first and second indicators are used to provide a resulting indicator. In an example, the resulting indicator is a value of a parameter (e.g. heart rate) at a particular activity level, or a characteristic (e.g. a slope, offset, difference, differential, or integral) of a relationship between two or more detected parameters, such as respiratory rate, tidal volume, and/or activity level, for example. In an example, interpolation of data is also used to provide a resulting indicator. In an example, the resulting indicator is an indicator of a shift in a relationship between respiration rate and tidal volume. FIG. 10, for example, shows a shift in a relationship between stroke volume and heart rate. FIG. 14 shows a shift in the relationship between respiration rate and tidal volume. In an example, an upward shift in the relationship between respiration rate and tidal volume is indicative of stiff lungs due to edema and/or heart failure decompensation.

In an example, if the first steady-state physical activity level and second steady-state physical activity level correspond to the same bin, the first and second indicators are combined to produce a combined indicator, which is used to provide the resulting indicator. In an example, if the first steady-state physical activity level and second steady-state physical activity level correspond to different bins, the first and second indicators correspond to different activity levels, as represented for example by points A and B in FIG. 7A.

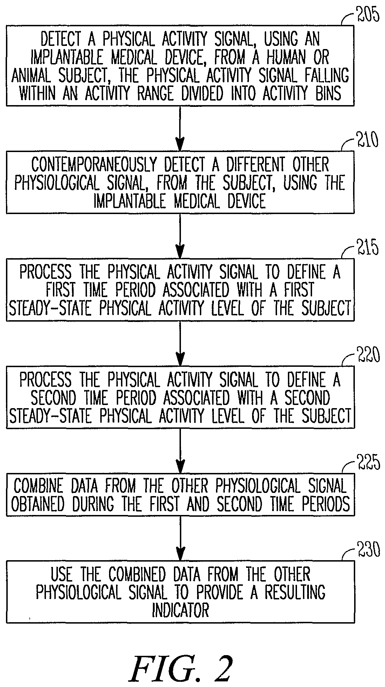

FIG. 2 is a flow chart that illustrates an example method in which physiological data is aggregated by activity level. At 205, a physical activity signal is detected from a human or animal subject using an implantable medical device. In an example, the activity range of an accelerometer or other sensor is divided into 20 activity bins. At 210, a different other physiological signal is contemporaneously detected from the subject using the implantable medical device. In an example, the different other physiological signal is detected using a sensor and is indicative of a parameter such as an intrinsic electrical heart signal, respiration, blood pressure, stroke volume, tidal volume, or venous blood oxygen saturation. In an example, two or more different other physiological signals are detected. At 215, the physical activity signal is processed to define a first time period associated with a first steady-state physical activity level of the subject. Processing the physical activity signal includes identifying for the first time period a first beginning time at which the physical activity of the subject is deemed to be at steady state at the first steady-state physical activity level, and a first ending time at which the subject leaves the first steady-state physical activity level. The first steady-state physical activity level corresponds to an activity bin. In an example, the processing includes comparing the activity bin to a previous bin and/or sequence of bins to determine one or more differences and/or trends in activity levels or bins corresponding thereto.

At 220, the physical activity signal is processed to define a second time period associated with a second steady-state physical activity level of the subject. The processing includes identifying a second beginning time at which the physical activity of the subject is deemed to be at steady state at the second steady-state physical activity level, and a second ending time at which the subject leaves the second steady-state physical activity level. The second steady-state physical activity level of the subject corresponds to the same activity bin as the first steady-state physical activity level of the subject.

At 225, data from the other physiological signal obtained during the first and second time periods is combined. In an example, the data is combined to determine aggregate average. A first average is determined for the first time period, a second average is determined for the second time period, and then the averages are combined to provide an aggregate average. In another example, a weighted averaged is determined, or the data from the first and second periods is combined and then averaged. At 230, the combined data from two or more activity levels is used to provide a resulting indicator, such as a slope or other differential, integral, difference, or other value that is indicative of a pathophysiological condition such as heart failure.

In an example, physiological signals such as respiratory rate and tidal volume are chronically monitored to monitor one or more physiological conditions. In an example, a second resulting indicator is obtained using data from a third and/or fourth steady state time period some time later (e.g. a week or more) than the first and second steady state time period. The resulting indicator obtained from the first and second time periods and the resulting indicator from the third and/or fourth time periods is used to monitor a physiological condition. In an example, a change in a relationship between respiratory rate and tidal volume is to monitor for stiff lungs due to edema or heart failure decompensation. For example, an upward shift in respiration vs. tidal volume during steady-state activity may be detected by monitoring physiological responses to steady state activity over the course of weeks, months, or years. FIG. 14 shows an upward shift in respiration v. tidal volume.

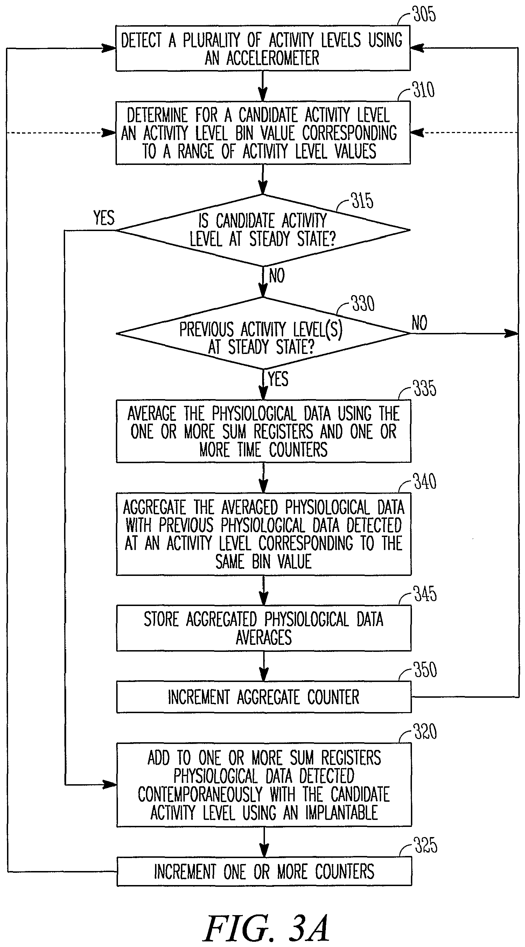

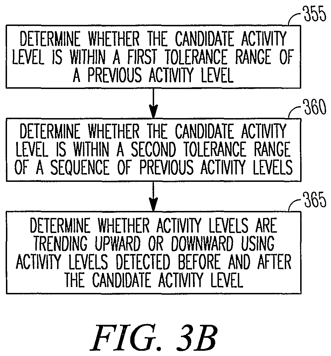

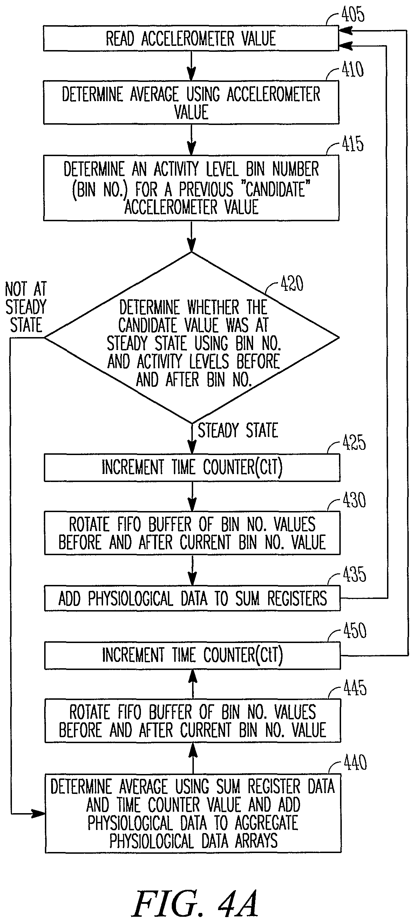

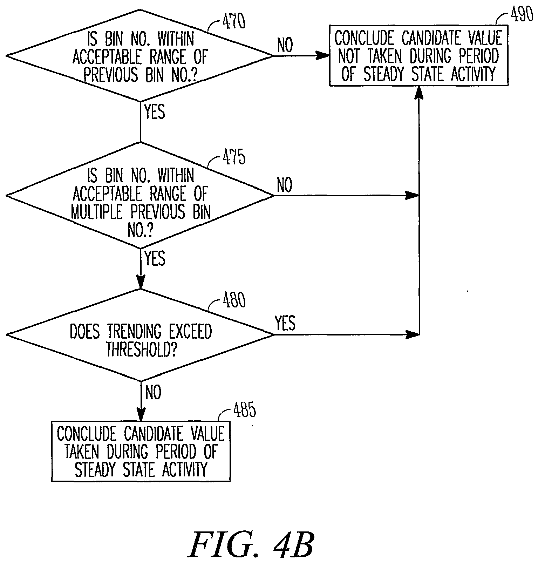

FIGS. 3A and 3B provide flow charts that illustrate additional example methods. At 305, a plurality of activity levels are detected using an accelerometer. In an example, an activity level is detected periodically by the accelerometer. In an example, an activity level is detected at least once every 10 seconds. At 310, an activity level bin value is determined for a candidate activity level. In an example, the candidate activity level is one of the detected activity levels. Alternatively, the candidate activity level is an average of one or more detected activity levels. The bins value corresponds to a range of activity levels. In an example, a bin value corresponds to a 10 mG range. For example, a first bin corresponds to 0-10 mG, a second bin corresponds to 10 - 20 mG, etc. In an example, the bins overlap (e.g. 9 to 21, 19 to 31, 29 to 41, etc.) At 315, it is determined whether the candidate level is at steady state. In an example, activity levels detected before and after the candidate activity level are used to determine whether the candidate activity level occurred during a period of steady state activity. FIGS. 4A and 4B show an example method of determining whether a candidate activity level is at steady state.

FIG. 3B illustrates an example method of determining whether the candidate activity level occurred during a period of steady state activity. At 355, it is determined whether the candidate activity level is within a first tolerance range of one or more previous activity levels. In an example, it is determined whether the candidate activity level is within the first tolerance range of the immediately preceding activity level, i.e. the last data point before the candidate activity level. In an example, the first tolerance range is defined in terms of data values, e.g. 20 - 30 mG. Alternatively the range is a number of activity bins, e.g. in the same bin, or within one bin. For example, if the range is one bin and the candidate activity level is in bin seven, the candidate activity level is deemed out of steady state if the bin value for the previous activity level is greater than eight or less than six. At 360, it is determined whether the candidate activity level is within a second tolerance range of a sequence of previous activity levels. In an example, the second tolerance range is larger than the first tolerance range, and a greater variance in activity level is tolerated for the sequence of activity levels than for the previous activity level. In an example, the second tolerance range is 10-40 mG, or alternatively the second tolerance range is two bins. At 365, it is determined whether activity levels are trending upward or trending downward, using activity levels detected before and after the candidate activity level. Trending analysis allows for identification of non-steady state periods where candidate activity levels fall within bin ranges but demonstrate rising or falling activity. Using activity levels detected both before and after the candidate activity level allows for better analysis of the candidate activity level in context than if only preceding activity levels were used.

Returning now to FIG. 3A, if the candidate activity level is at steady state, at 320 physiological data detected contemporaneously with the candidate activity level using an implantable medical device is added to a sum register. At 325, a counter is incremented. For example, if the candidate activity level is the ninth detected activity level in a period of steady state activity, the counter is incremented from eight to nine. As explained below, the counter is later used at 335 to determine an average, using the summed physiological data, when the period of steady state activity ends (e.g. (summed data)/(counter value) = average). The method returns to 305 and additional activity levels are detected.

Returning to operation 315, if the candidate level is not at steady state, at 330, it is determined whether the candidate value represents the end of a period of steady state activity by determining whether one or more previous activity levels are at steady state. If the one or more previous activity levels were not at steady state, i.e. if the candidate value does not represent the end of a period of steady state activity, the method returns to 305. If the previous activity level was at steady state, at 335, an average is determined using the data in the sum register and the counter. In an example, the value in the sum register is divided by the counter value to determine the average physiological data value for the steady state period. At 340, the averaged physiological data is aggregated with previous physiological data detected at an activity level corresponding to the same bin value. Aggregated physiological data average is stored at 345, an aggregate counter is incremented at 350, and the process returns to 305. In an example, the aggregated physiological data is used to provide a resulting indicator. It is understood that the processing of the data does not necessarily occur as the data is collected. In an example, the data is collected first, and then processed, in which case the method would return to 310 after 325, 330, and/or 345.

FIGS 4A and 4B are flowcharts that illustrate an example algorithm in more detail. The algorithm accumulates aggregated physiological data correlated to steady state activity. At 405, a value is read from an accelerometer. At 410, an average of a plurality of detected activity levels is determined. In an example, the average is a "BoxCar average." In an example, the box car average is an average of a number of sequential accelerometer values. In an example, accelerometer values are included in the BoxCar average for a number of averaging operations, and then purged from the BoxCar average. In an example, a BoxCar average determined at 410 includes seven values - three values preceding a candidate value and three values succeeding the candidate value (e.g. 14, 16, 19, XX, 18, 17, 18, wherein, XX is the candidate value.)

At 415, an activity level bin number (binNo) for a candidate accelerometer value is determined. The candidate value is a value for which a steady state analysis is conducted using a number of values taken before and after the candidate value. In an example, the candidate value is an average of a number of detected accelerometer values. A number of bins corresponding to ranges of activity levels are defined. In an example, bin 1 includes activity levels from 0-4 mG, bin 2 includes activity levels from 4-8 mG, bin 3 includes activity levels from 8-12 mG, etc. In an example, the activity level bin number for the candidate value is determined by dividing the candidate value by a bin width value (e.g. 4 mG) and truncating the quotient. For example, if the bin width value is 4, the binNo for activity levels 8, 9, 10, or 11 would be 2 and the binNo for activity levels 12, 13, 14, or 15 would be 3.

At 420, a steady state analysis is conducted. In an example, one or more algorithms are performed to determine whether the candidate value was taken during a period of steady state activity. FIG. 4B illustrates an example steady state method analysis method that uses the binNo for the candidate value and for values before and after the candidate value. At 470, the binNo for the candidate value is compared to the previous binNo value. If the binNo value is not within an acceptable range of the previous binNo value (e.g. 1 bin or 2 bins), it is concluded at 490 that the candidate value was not taken during a period of steady state activity. If the binNo value is within an acceptable range of the previous binNo value, at 475, the binNo is compared to a plurality (e.g. six) preceding binNo values. If the binNo is not within an acceptable range of the preceding binNo values (e.g. 3 bins), it is concluded that the candidate value was not taken during a period of steady state activity. In an example, the acceptable range for the plurality of preceding binNo values is larger than the acceptable value for the previous binNo value. If the binNo value is within an acceptable range of the plurality of preceding binNo values, at 480 binNo values before and after the candidate binNo value are analyzed for trending. Alternatively, only values occurring before, or after, the candidate value are used in the trending analysis. If the binNo values do not exhibit trending, or if the binNo values exhibit trending below a threshold, it is concluded at 485 that the candidate value corresponds to a period of steady state activity. In the example illustrated in FIG. 4B, if one of the operations 470, 475 returns a No value, i.e. if the operation indicates that the candidate value was not taken during a period of steady state activity, the succeeding operations (e.g. operation 480) are not performed, thereby avoiding unnecessary processing and conserving processing power and/or battery energy. Alternatively, all three operations are performed regardless of the output of operation 475, 480. It is also understood that the operations 470, 475, 480 could be performed in a different order. For example, the trending analysis operation 480 could come first instead of last.

Returning to FIG. 4A, if the candidate value is determined to correspond to a period of steady state activity, at 425, a time counter is incremented. At 430, a buffer of previous binNo values is rotated. In an example, the buffer is a first in-first out (FIFO) buffer. The buffer of previous binNo values are used in operations 470, 475, and 480 in FIG. 4B. At 435, physiological data is added to sum registers. In an example, the physiological data includes one or more of heart rate (HR), tidal volume (TV), respiratory rate (RR), total minute ventilation (MV), pulmonary artery diastolic pressure (Ppa, dias), mixed venous blood oxygen saturation (Ven Sat), cardiac output (CardOut), and stroke volume of the heart (SV). It is understood that in alternative examples, some of the operations in FIG. 4A could be performed in a different order. For example, operations 425, 430, and 435, could occur in a different order or could occur simultaneously.

Referring again to FIG. 4A, if, at 420, the candidate value is determined to be during a non-steady state period, at 440, physiological data in the sum registers (if any) is processed (e.g. averaged) and added to physiological data arrays that aggregate data. In other words, the occurrence of a non-steady state value after a period of steady state activity triggers the aggregation of data in the sum registers into an aggregate data array. At 445, the FIFO buffer of binNo values before and after the current binNo value is rotated. At 450, the time counter is incremented.

FIGS. 5A is a schematic illustration of a medical device 500, lead 520, and electrodes 505, 510, 515 that are located at a location in a body, such as in, on or around a heart 501. The electrodes 505, 510, 515 are coupled to conductors that extend through the lead 520 and connect a header 530 on the medical device 500. Another electrode 525 is located on the medical device housing 535. In an example, the electrodes an antitachyarrhythmia therapy such as a defibrillation signal. In an example, electrodes 515, 525 or additional electrodes are used for sensing a physiological parameter, such as an intrinsic electrical heart signal.

FIG. 5B is a schematic illustration of an example configuration of the medical device 500 of FIG. 5A, electrodes, and sensors. The electrodes 505, 510, 515, 525 are coupled to a pulse generator 540 and optionally also to sensing circuitry 545, which are coupled to an analysis circuit 550. The analysis circuit 550 receives a signal from an activity sensor 555, which may be an accelerometer, for example, and at least one additional signal detected by one of the electrodes 505, 510, 515, 525 or another sensor, such as pressure sensor 560, or venous blood oxygen saturation sensor 565. In an example, a signal from a posture sensor 570 is also used in combination with the activity sensor 555 to detect periods of steady state activity.

FIG. 6B shows a table of example data values arranged according to steady state activity levels. These data would be obtained, for example, using the activity and physiological data maintained in the aggregate data array at 440 in FIG. 4A. The first column lists bin numbers (Bin No.) The second column lists activity level ranges in milli-G's (thousandths of gravitational acceleration (3.81 m/s²). In the example table of FIG. 6B, the bin width is 25 mG. An accelerometer typically displays a value less than 25 when the patient is resting in a sitting, lying, or standing position. Accelerometer values of 25-50 typically correspond to walking slowly on a level surface, for example. Values higher than 50 correspond to faster walks, for example. Values in the 75-100 typically correspond to moderate exertion, such as light jogging. Accelerometer values greater than 100 typically refer to more strenuous exertion.

More or fewer bins could be used in other examples. In an example where more bins are used, each bin has a width of 5 mG. In another example, the bin width varies across the range of bins. In an example, a non-linear set of bins is used (e.g. 0 to 4 mG, 4 to 16 mG, 16 to 64 mG, 64 to 128 mG).

Referring again to FIGS. 6, columns three to eleven in the table provide data that relating to physiological responses to steady state activity for the various activity levels listed in the second column: The third column is the heart rate in beats per minute (BPM). The fourth column is the tidal volume (TV) in liters. The fifth column is the respiratory rate in breaths per minute (BPM). The sixth column is the minute ventilation in liters per min (L/min). The seventh column is the diastolic pressure of the pulmonary artery in millimeters of mercury (mm Hg). The eighth column is venous blood oxygen saturation. The ninth column is cardiac output in liters per minute (L/min). The tenth column is the stroke volume of the heart in milliliters (ml). The last column is the number of steady state minutes observed for each activity level bin.

Example methods and apparatus in accordance with this disclosure can provide some or all of the types of physiological data shown in FIG. 6B, depending on the specific embodiment and configuration. In an example, each cell of data in columns 3-9 of the table represents an average of a detected physiological parameter detected during one or more periods of steady state activity corresponding to the accelerometer range for that row. In an example, values for physiological variables are included in the reported average each time the activity sensor returns to a given bin range for a length of time that indicates steady state activity. In an example, data detected during a period of steady state activity is combined to provide a period average for the steady state period. If the activity level returns to the steady state bin, an aggregate average, i.e. an average of the averages, is computed and provided in the table.

In an example, the total time spent at each level of activity is also tracked (see column 11 in FIG. 6B), which allows estimation of total exertion during the time period over which averaging continues. In an example, data is added to the table over a defined time period defined, such as 24 hours, 12 hours, or shorter periods, or over periods activated by the patient. In another example, data collection is initiated at the beginning of an exercise test. A typical exercise test lasts about 20 minutes. In an example, collection of data is initiated by the subject in whom the medical device is implanted.

In an example, data tables are stored or archived, and physiological data obtained at one time point is compared against data obtained at other time points.

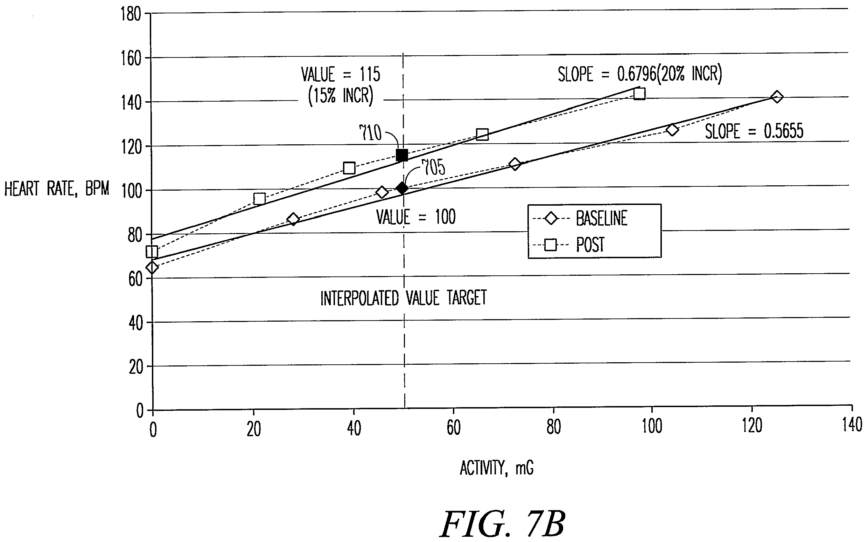

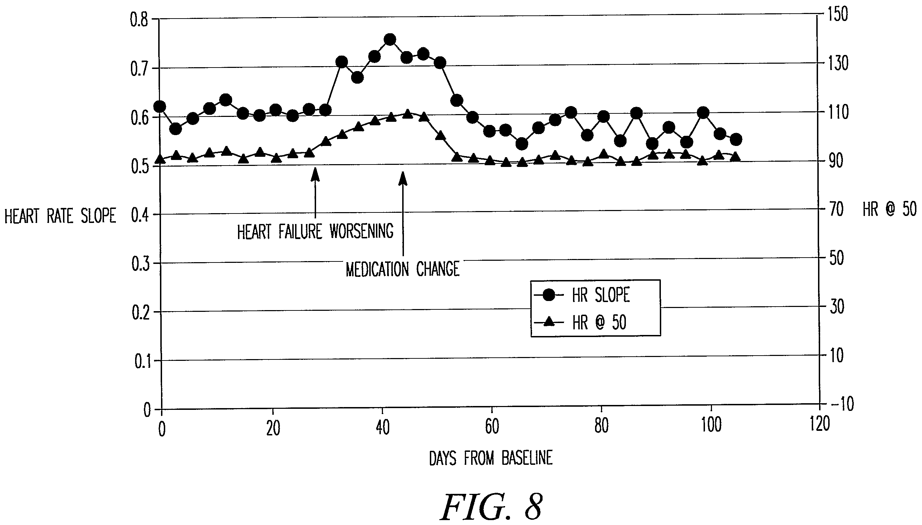

Data gathered using the present methods and apparatus, such as the example shown in the table of FIG. 6B, is usable to monitor and/or diagnose one or more physiological conditions. FIGS. 7A to 17 are graphs that show examples of physiological data that are obtainable through examples of the presently described apparatus and methods. Example resulting indicators are obtainable from the graphs and/or data shown in FIGS. 7A-17. In some examples, graphing physiological data for a particular time point provides a useful resulting indicator. In FIG. 7A, for example, the slope of a heart rate vs. activity plot for a sample period (e.g. 24 hours) can be used as a resulting indicator. In other examples, a change in a resulting indicator is observable from data and/or graphs for multiple points in time, such as the shift of the two lines shown in FIG. 7B. Observed changes in physiological responses to steady state activity can indicate development or progression of a pathophysiological condition, such as heart failure. In another example, an indicator is monitored using a graph (or the underlying data) showing a parameter plotted against a time axis (e.g. FIG. 8).

In the graphs of FIGS. 7B and 9-17, data is provided for a Baseline time period, and for a later "Post" time period. While the present discussion focuses on physiological variables relating to the cardiac and/or respiratory systems, additional variables could be added or substituted to extend observations and analysis to other organ systems, such as kidneys, leg muscles, or autonomic nervous system.

To understand the significance and utility of the information obtainable through the presently described algorithms and represented in FIG. 7A-17, it may be useful to understand some principles of operation of the cardiovascular system. The heart and lungs together supply working muscles with the extra oxygen required by increasing activity. The amount of oxygen delivered to the tissues per minute is determined by the product of the heart rate, the stroke volume of the heart, and the oxygen carrying capacity of the blood (HR x SV x Ca,O2). As activity increases, controller mechanisms in the body cause increases in both heart rate and stroke volume. The amount of increase in each of these variables for a given increase in activity depends on multiple factors including health of the heart, fitness of the individual, and pharmacologic therapy. Diseased hearts have a reduced ability to increase stroke volume. Because of this reduced ability to increase stroke volume, as the body tries to increase cardiac output required by a period of activity, the heart rate of a diseased heart for a given activity will be higher and/or rise more quickly than the heart rate for a healthy heart. Thus, a change in the rate of increase of either heart rate or stroke volume in response to activity is an indicator of a change in either health and/or treatment status of the patient.

Referring now to FIG. 7A, a graph of heart rate against activity is shown. In a healthy person, heart rate typically increases linearly with external work intensity. Because activity as sensed by an accelerometer in implanted devices is related to external work intensity, the increase in heart rate plotted against activity is typically linear. The heart rate increases because of stimuli originating from working muscles that tell the central nervous system of a need to increase oxygen delivery to working muscles. The rate of increase depends on the fitness level of the person (more fit individuals will have a smaller increase in heart rate than less fit people), disease condition of the heart, and pharmacological therapy. A patient with diastolic heart disease will have reduced ability to increase stroke volume, and the heart rate at any given activity will tend to be higher. A person on beta blocker therapy will likely have a reduced heart rate for a given activity because the beta blocker drug acts directly on the nerves endings in the heart that cause heart rate to increase during activity. The heart rate or rate of change of heart rate is usable, for example, as an indicator of fitness, the progression of disease, and/or the effectiveness of a therapy.

Referring now to FIG. 7B, two sets of data of shown on the graph, one for a "Baseline" measurement and one for a "Post" measurement. In an example, the Post measurement represents data collected after some time has elapsed from the "Baseline", or after some intervention such as change in medication. In an example, a curve-fitting operation is used to determine a curve from the data. In FIG. 7B, a line has been determined for the baseline data and for the Post data. Interpolated values 705, 710 are also shown for each data set for an activity level of about 50 mG. In an example, a difference between heart rates at the 50 mG activity level for the Baseline and Post measurements is used as a resulting indicator. In an example, a difference between the interpolated values is used as a resulting indicator. FIG. 7B shows a difference of 15 beats per minute at the interpolated values for 50 mG. In another example, an average difference over a range of values is used as a resulting indicator. In an example, the difference between the two lines is integrated over a range of activity levels and the integral is used as a resulting indicator. In another example, the slope of one or both lines is used as an indicator. The slopes of the lines are shown numerically on FIG. 7B. In another example, the change in the slope (e.g. slope of Post line minus slope of Baseline line) is used as a resulting indicator.

FIG. 8 is a plot of heart rate at an activity level of 50 mG. FIG. 8 also shows periodic values for the first derivative (slope) of the heart rate against activity, i.e. the slope of the heart rate data from the graph shown in FIG. 7B. In the example shown in FIG. 8, the heart rate and heart rate slope are plotted against Days from Baseline measurement, with two clinically significant events indicated. An increase in the heart rate slope and the HR @ 50 mG (e.g. around days 30-45) indicates that the patient's heart condition is deteriorating. After treatment, both values dropped around day 45.

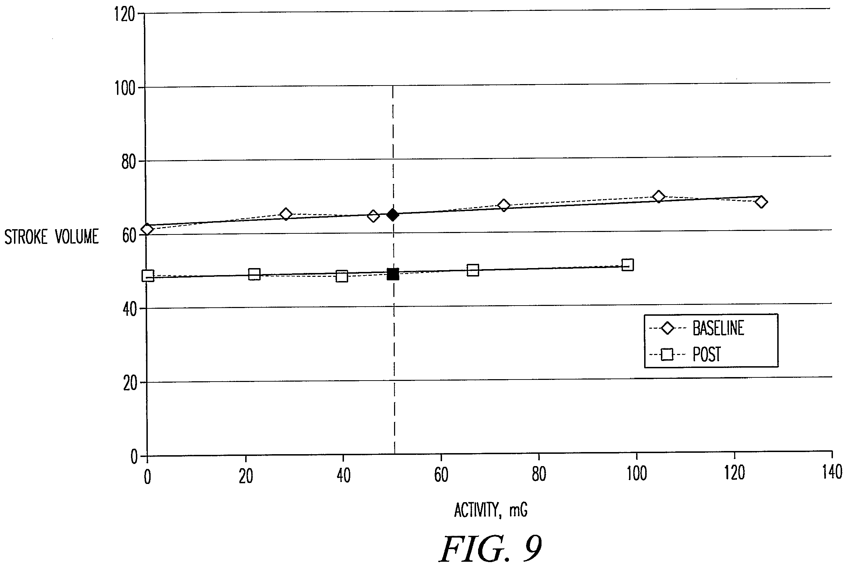

FIG. 9 is a plot of stroke volume vs. activity. The rate of increase (i.e. slope) of stroke volume with increasing activity is lower in the Post condition than the baseline condition, indicating that heart condition has deteriorated since the baseline measurements.

FIG. 10 is a plot of stroke volume (in milliliters) against heart rate (in beats per minutes). The disease condition of the heart may be made more evident by plotting heart rate and stroke volume together as shown in FIG. 9 rather than individually against activity. In an example, this plot amplifies a change in heart function compared with plots of either variable alone against activity. In an example, a reduction in SV at a heart rate of 100 beats per minute indicates a reduction in the heart's ability to pump blood. FIG. 10 shows interpolated values for stroke volume at 100 beats per minute. The reduction in stroke volume in the "Post" condition compared to Baseline indicates a change in ability of the heart to pump blood.

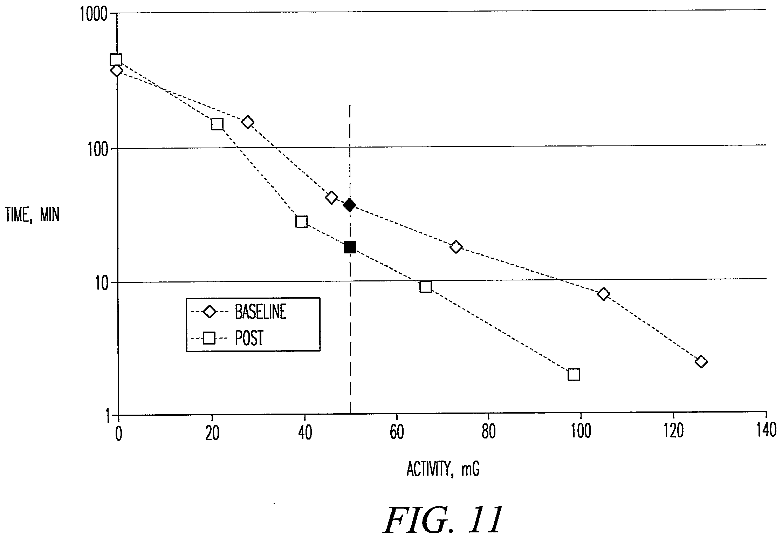

FIG. 11 is a logarithmic plot that shows time spent at a given activity level. The higher time spent at zero activity during the Post measurement period than the Baseline period suggests deteriorating heart condition. The reduced time spent at higher levels of activity during the Post period compared to the Baseline period also suggests deteriorating heart condition. An interpolated time value at an activity level of 50 mG is indicated. In an example, a difference between interpolated values, or between a measured value and an interpolated value, is used as a resulting indicator. In the example shown, the interpolated value of time at 50 dropped by slightly over half, from 37 min to 18 minutes. Thus, time at 50 may be a particularly sensitive measure of patient well being.

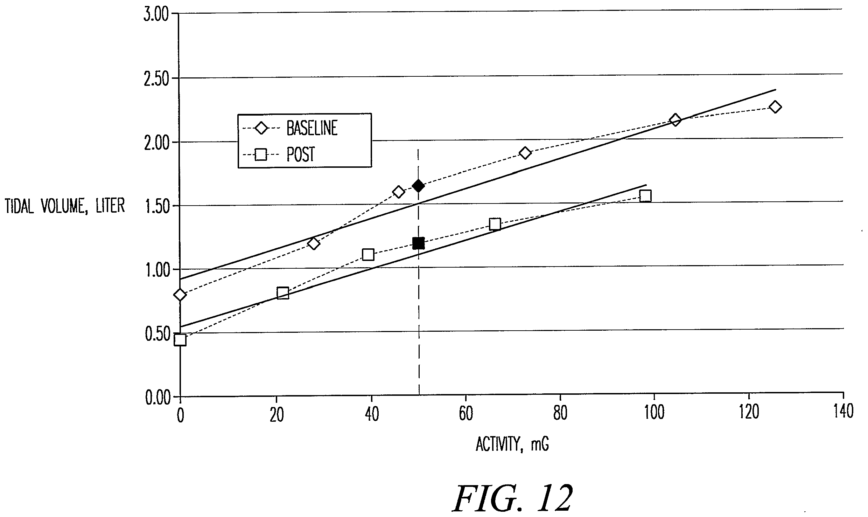

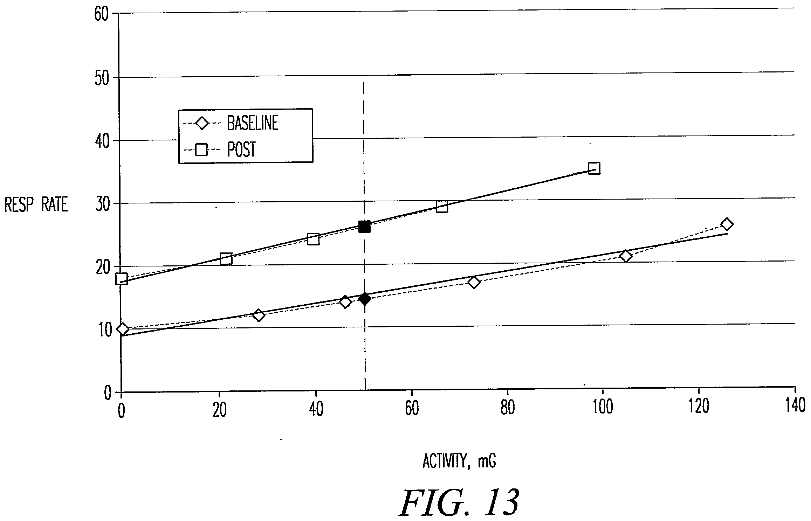

FIGS. 12 shows tidal volume plotted against activity. FIG. 13 shows respiration rate plotted against activity. As activity increases, increasing HR and SV to deliver more oxygen to the tissues, the lungs must bring in more fresh air to keep the incoming blood saturated with oxygen and to keep the blood at a normal level of Carbon Dioxide (CO2) when the blood leaves the lungs. In a fashion similar to control of heart rate and stroke volume, the body increases respiratory rate (RR) and tidal volume (TV) as activity increases to control oxygen saturation and CO₂ levels in arterial blood. The total ventilation is the product of respiration rate and tidal volume (MV = RR x TV). The amount of increase in minute ventilation (MV) required by the body is dictated by the health of the lungs and by efficiency of the heart in delivering blood flow to the lungs. It is known, for instance, that people with congestive heart failure need larger increase in MV for a given activity because the lungs become less efficient when cardiac output is reduced. In addition, stiff lungs affect the pattern of increasing respiration rate and tidal volume to reach a given target ventilation. When congestive heart failure becomes very severe, lungs become stiffer due to accumulated fluid in the interstitial spaces, resulting in a pattern of higher respiration and lower tidal volume compared to healthy lungs. Thus, tracking the pattern of change in respiration rate and tidal volume in response to steady state activity provides an indicator of the progression of congestive heart failure.

In an example, a change in minute ventilation during activity is tracked using a sensor that measures changes in electrical impedance across the lungs. With each tidal breath, impedance increases as the lung expands and decreases as they empty. Both relative tidal volume and respiration rate are obtainable from this signal. Relative tidal volume refers to a ratio of a present tidal volume to a baseline or maximum tidal volume. In another example, an actual quantified tidal volume is determinable from an external device that measures airflow directly (using a pneumotachograph) or measures chest wall dimensions.

Referring again to the graphs of tidal volume and respiration rate against activity shown in FIGS. 12 and 13, in the Post condition, for any given activity level, the tidal volume is higher than in the baseline condition, and the respiration rate is lower than in the baseline condition. This combination commonly occurs as lungs become mechanically stiffer due to accumulation of water caused by backup of fluid when diastolic pressure in the pulmonary artery increases in heart failure. In an example, one or both of these shifts is indicative of changing lung mechanics.

FIG. 14 shows respiratory rate (RR) plotted against Tidal Volume (TV). The upward shift in the curve in the "post" condition compared to Baseline indicates a change in lung mechanics. In an example, the upward shift indicates stiff lungs due to edema or heart failure decompensation. In an example, the graph of respiration rate against tidal volume amplifies changes in lung mechanics that are also detectable in the graphs of FIGS. 12 and 13.

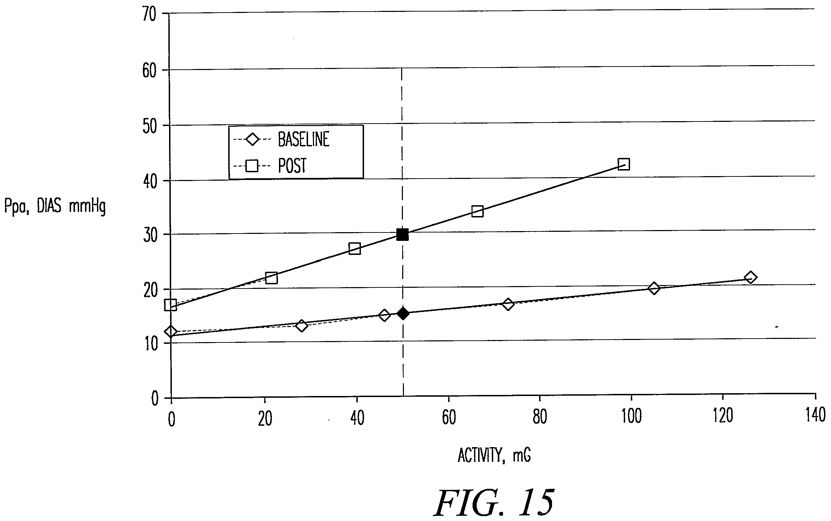

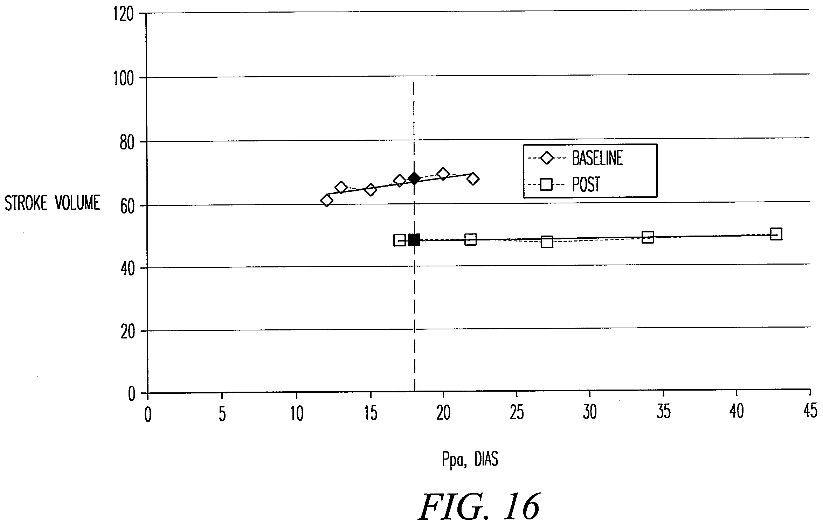

FIG. 15 shows diastolic pressure in the pulmonary artery plotted against activity. FIG. 16 shows stroke volume plotted against pulmonary artery diastolic pressure. The heart cycle is split into two phases, an active contraction phase, called systole, and a passive filling phase, called diastole. During diastole, the heart fills with blood and during systole, the filled heart contracts to force the blood out into the arterial system. In diastole, blood flowing back to the heart enters the heart by largely passive means, and as the heart fills with blood the pressure inside rises. The amount of rise in pressure is one indication of heart health: a healthy heart can expand with little change in pressure, whereas a diseased heart is stiff and requires larger pressures to fill. In an example, a sensor detects diastolic pressure in the pulmonary artery, and the pressure needed to fill the heart is tracked along with stroke volume. A shift in the relationship between stroke volume and diastolic pressure in the pulmonary artery indicates a change in the stiffness of the heart.

Referring again to FIG. 15, pressure in the pulmonary artery measured at end of diastole (Ppa, dias) is plotted against activity for Baseline and Post conditions. The increased pressure at all activities in the Post measurements indicates a change in cardiac mechanics or fluid status. In an example, an increase in pulmonary artery diastolic pressure indicates fluid retention and heart failure decompensation. In another example, an upward shift in slope indicates a change in stiffness of the heart.

FIG. 16 shows stroke volume plotted against diastolic pressure in the pulmonary artery for baseline and post conditions. A drop in the stroke volume for all pulmonary artery diastolic pressure values indicates reduced compliance (increased stiffness) of the heart. A reduction in the slope of the lines also indicates reduced compliance of the heart. In an example, the graph and data shown in FIG. 16 is used to track changes in heart condition. In another example, the graph and/or data shown in FIG. 16 is used to diagnose a heart conditions. A rise in pulmonary artery diastolic pressure does not necessarily indicate a stiff heart. For example, pulmonary artery diastolic pressure will also rise if arterial pressure is too high (systemic hypertension) for the left ventricle to completely empty during systole. A healthy heart pumping against arterial hypertension would likely show higher SV in the graphs of FIGS. 15 and16.

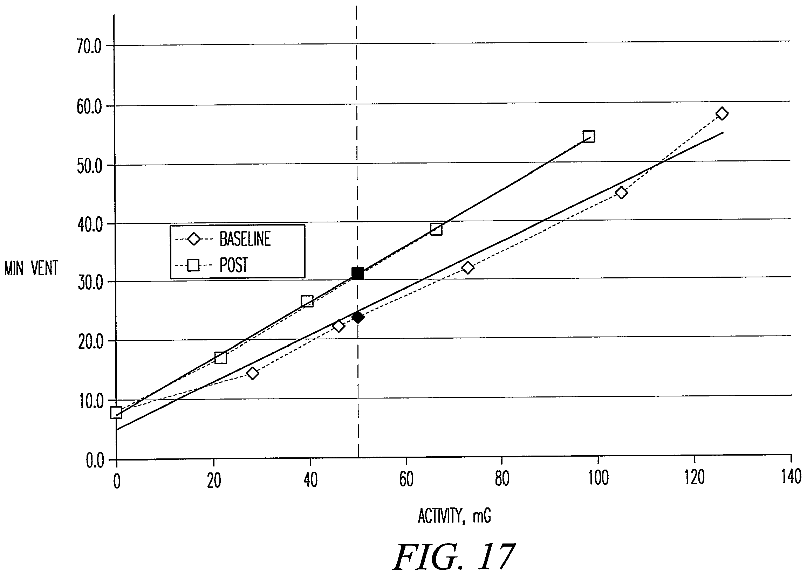

FIG. 17 shows cardiac output plotted against activity. Cardiac output is the product of stroke volume and heart rate (CardOut= SV x HR). In normal individuals, this relationship is typically constant, and does not vary with conditioning level of the subject. In an example, a change in the CardOut vs. activity relationship (e.g. a downward offset of the line in FIG. 17) is used as an indicator of heart disease. In another example, a reduction of the slope of CardOut vs. activity relationship indicates progression of heart disease. In an example, one or more sensors measures stroke volume or cardiac output.

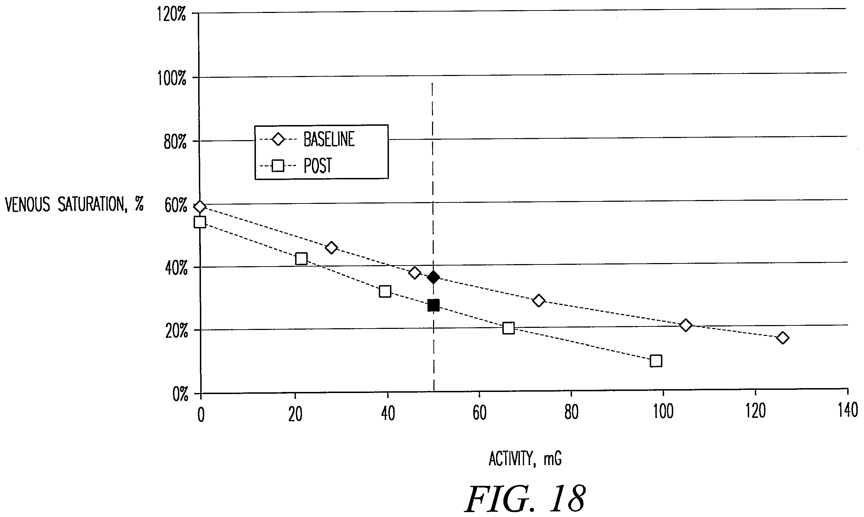

In another example, venous oxygen saturation level of blood is used as an indicator of cardiac status. FIG. 18 is a graph that shows venous oxygen saturation (VenSat) plotted against activity for Baseline and Post timeframes. The reduction in VenSat at activities above the resting level at the Post time indicates an inability of the heart to increase Cardiac Output to support tissue oxygen demands. As the heart increases its output with activity, increased blood flow goes to working muscles, which extract oxygen to support increased metabolism. The amount of extraction is determined by a mass balance relationship called the Fick Equation, VO₂ = CardOut x (CaO₂ - CvO₂), where VO₂ is oxygen consumption, CardOut is the cardiac output and CaO₂- CvO₂ is the difference in oxygen content of arterial and mixed venous blood, respectively. Re-arranging the Fick equation shows that this difference reflects the balance between VO₂ and CardOut: (CaO₂- CvO₂) = VO₂/CardOut. If CardOut is low compared to VO₂ the CaO2- CvO₂ difference must be larger, as the working muscles need to extract more oxygen per unit of blood when blood flow is inadequate. Because the CaO₂ is relatively constant, the CaO₂- CvO₂ difference can only widen by reducing CvO₂. The content of oxygen in the blood is the product of the degree of O₂ saturation of the Hemoglobin molecule, VenSat, times blood content of hemoglobin. Because blood content of hemoglobin also stays constant, variations in VenSat will reflect the balance of VO₂ and CardOut. Conditions in which CardOut is insufficient will cause VenSat to drop, as illustrated in FIG. 18. A change in the venous blood oxygen saturation at a steady state activity level is usable as an indicator of heart condition. In an example, a reduced venous blood oxygen saturation at a given acitivity level indicates a reduced ability of the heart to supply oxygen to tissues and a progression of heart disease.

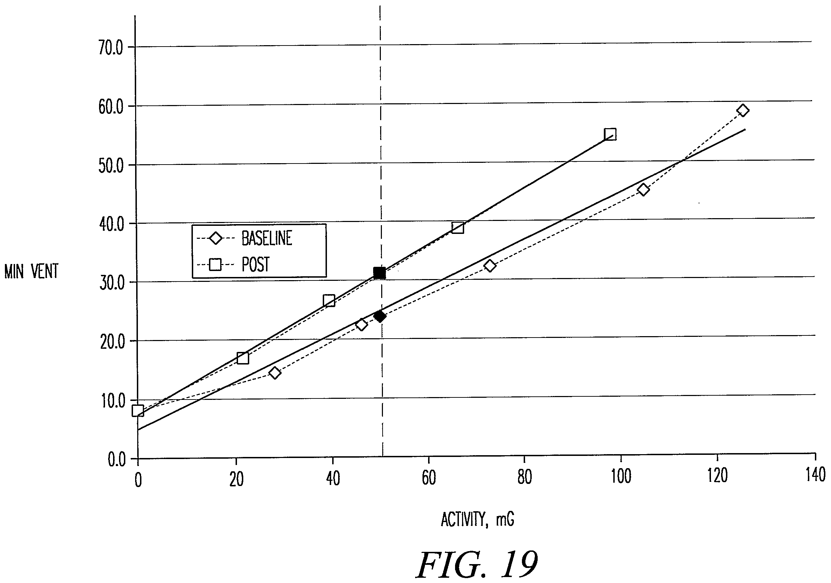

FIG. 19 is a graph that shows minute ventilation plotted against activity. In an example, an increase in the slope of minute ventilation vs. activity or an increase in minute ventilation at a particular acitivity level indicates abnormal lung function, such edema.

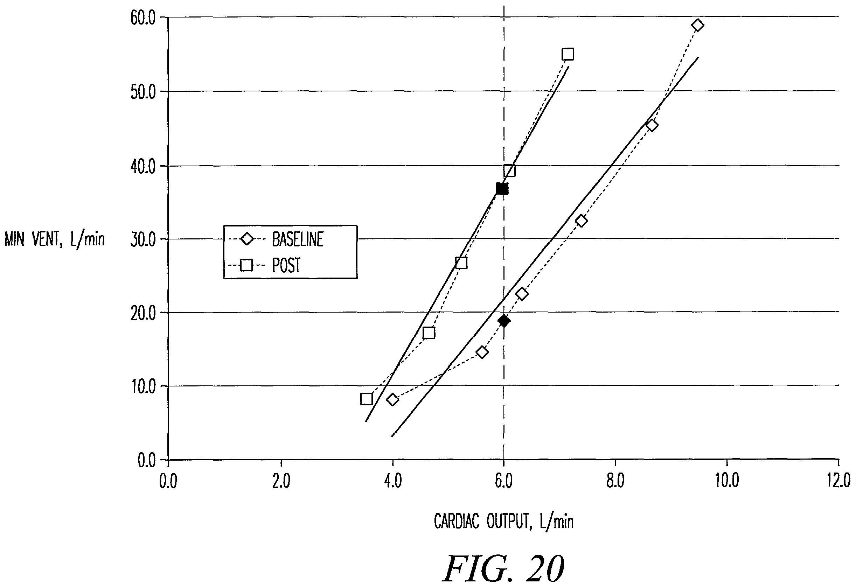

FIG. 20 is a graph that shows minute ventilation plotted against cardiac output. In an example, increasing minute ventilation for a given cardiac output indicate deteriorating heart condition.

The above specification, examples and data provide a complete description of the manufacture and use of the composition of the invention. Since many embodiments of the invention can be made without departing from the scope of the invention, the invention resides in the claims hereinafter appended.

## Claims

1. A system comprising:
an implantable activity sensor (555), configured for detecting a physical activity signal from a human or animal subject;
an implantable first other physiological sensor (505, 510, 515, 525, 560, 565), configured for detecting a first other physiological signal from the subject, the first other physiological signal being different from the physical activity signal;
a signal processor (550), configured to process the physical activity signal to define a first time period associated with a first steady-state physical activity level of the subject, including identifying for the first time period a first beginning time at which the physical activity of the subject is deemed to be at steady state at the first steady-state physical activity level, and a first ending time at which the subject leaves the first steady-state physical activity level, the signal processor obtaining a first indicator by using data from the first other physiological signal obtained during the first time period, the signal processor processing the physical activity signal to define a second time period associated with a second steady-state physical activity level of the subject, including identifying for the second time period a second beginning time at which the physical activity of the subject is deemed to be at steady state at the second steady-state physical activity level, and a second ending time at which the subject leaves the second steady-state physical activity level, the signal processor obtaining a second indicator by using data from the first other physiological signal obtained during the second time period, the signal processor using the first and second indicators to provide a first resulting indicator,
wherein the processor (550) being configured for defining a first activity bin for a range of activity levels comprising the first steady state physical activity level, and wherein the system further comprises an implantable accelerometer (555), configured for detecting a plurality of activity levels, the processor (550) including activity level bin counters and physiological data sum registers, the processor configured for determining for a candidate activity level an activity level bin value corresponding to a range of activity level values, and for determining whether the candidate activity level was at steady state using bin values from activity levels detected before and after the candidate activity level and, when the candidate activity level is determined to be at steady state, then adding to one or more of the sum registers physiological data detected contemporaneously with the candidate activity level, and incrementing one or more of the counters, and when the activity level is determined to be not at steady state and a previous activity level was at steady state, then averaging the physiological data using the one or more sum registers and one or more of the counters, and wherein the processor is further configured for aggregating the averaged physiological data with previous physiological data detected at an activity level corresponding to the same bin value, and wherein the processor includes or is coupled to a memory for storing aggregated physiological data averages.

2. The system of claim 1, in which the processor (550) includes a second activity bin for a range of activity levels comprising the second steady-state physical activity level, wherein the first and second activity bins correspond to different physical activity levels.

3. The system of claim 1, in which the second steady-state physical activity level corresponds to the same first activity bin as the first steady-state physical activity level.

4. The system of claim 3, wherein the second time period occurs after the first time period, and the second indicator combines data from the first other physiological signal obtained during the second time period with data from the first other physiological signal obtained during the first time period.

5. The system of claim 4, wherein the processor (550) is configured to process the physical activity signal to define a third time period associated with a third steady-state physical activity level of the subject, including identifying for the third time period a third beginning time at which the physical activity of the subject is deemed to be at steady-state at the third steady-state physical activity level, and a third ending time at which the subject leaves the third steady-state physical activity level, the third time period occurring between the first time period and the second time period, and obtaining a third indicator by combining data from the first other physiological signal obtained during the third time period.

6. The system of claim 1, further comprising an implantable second other physiological signal sensor, configured for detecting a second other physiological signal from the subject, and obtaining third and fourth indicators by using data from the second other physiological signal obtained during the respective first and second time periods, wherein using the first and second indicators to provide the resulting indicator includes using the first, second, third, and fourth indicators to provide the resulting indicator.

7. The system of claim 1, wherein the processor (550) is configured for obtaining the first indicator by using data from the first other physiological signal obtained during the first time period by averaging the data from the first other physiological signal obtained during the first time period.

8. The system of claim 1, wherein the resulting indicator includes an offset, a difference, a derivative, an interpolation, or an integral.

9. The system of claim 1, wherein the resulting indicator provides information about a relationship between heart rate and activity.

10. The system of claim 1, wherein the resulting indicator provides information about a relationship between stroke volume and activity or heart rate.

11. The system of claim 1, wherein the resulting indicator provides information about a relationship between at least two of respiratory rate, activity, and tidal volume.

12. The system of claim 1, wherein the resulting indicator provides information about a relationship between an intravascular pressure or venous blood oxygen saturation and activity level or one or more other physiological parameters.

13. The system of claim 1 comprising:
an implantable activity sensor (555), configured for detecting a physical activity signal from a human or animal subject, the physical activity signal falling within an activity range divided into activity bins;
an implantable first other physiological sensor (505, 510, 515, 525, 560, 565), configured for contemporaneously detecting a different first other physiological signal, from the subject;
a processor (550), configured for processing the physical activity signal to define a first time period associated with a first steady-state physical activity level of the subject, including identifying for the first time period a first beginning time at which the physical activity of the subject is deemed to be at steady state at the first steady-state physical activity level, and a first ending time at which the subject leaves the first steady-state physical activity level, the first steady-state physical activity level corresponding to an activity bin, the processor (550) configured for processing the physical activity signal to define a second time period associated with a second steady-state physical activity level of the subject, the second steady-state physical activity level of the subject corresponding to the same activity bin as the first steady-state physical activity level of the subject, including identifying for the second time period a second beginning time at which the physical activity of the subject is deemed to be at steady state at the second steady-state physical activity level, and a second ending time at which the subject leaves the second steady-state physical activity level, and the processor combining data from the first other physiological signal also obtained during the first and second time periods, and the processor determining a resulting indicator using the combined data from the first other physiological signal.

14. The system of claim 13, in which the processor (550) is configured for processing the physical activity signal to define a third time period associated with a third steady-state physical activity level of the subject, including identifying for the third time period a third beginning time at which the physical activity of the subject is deemed to be at steady state at the third steady-state physical activity level, and a third ending time at which the subject leaves the third steady-state physical activity level, the third steady-state physical activity level corresponding to an activity bin, and wherein the processor is configured for processing the physical activity signal to define a fourth time period associated with a fourth steady-state physical activity level of the subject, the fourth steady-state physical activity level of the subject corresponding to the same activity bin as the first steady-state physical activity level of the subject, including identifying for the fourth time period a fourth beginning time at which the physical activity of the subject is deemed to be at steady state at the fourth steady-state physical activity level, and a fourth ending time at which the subject leaves the fourth steady-state physical activity level, and wherein the processor is configured for combining data from the other physiological signal also obtained during the third and fourth time periods, and wherein the processor determines a second resulting indicator by using the combined data from the first other physiological signal obtained during the third and fourth time periods, and wherein the processor is configured for using the first and second resulting indicators to monitor a physiological condition of the subject.

15. The system of claim 13, wherein the processor (550) is configured for combining data from the first other physiological signal obtained during the first and second time periods by determining a first average of data obtained during the first time period, obtaining a second average of data obtained during the second time period, and combining the first average and the second average.

16. The system of claim 13, wherein the processor (550) is configured for determining the first resulting indicator using the combined data from the first other physiological signal by determining a heart rate, stroke volume, tidal volume, or respiration rate for a steady state activity level.

17. The system of claim 13, wherein the processor (550) is configured for determining the first resulting indicator using the combined data from the first other physiological signal by determining a venous blood oxygen saturation or pulmonary artery diastolic pressure for a steady state activity level.

18. The system of claim 1, wherein the processor (550) is configured for determining whether the candidate activity level was at steady state by determining whether the candidate activity level is within a first range of a previous activity level, determining whether the candidate activity level is within a second range of a sequence of previous activity levels, the second range larger than the first range, and determining whether activity levels are trending upward or downward using activity levels detected before and after the candidate activity level.

19. The system of claim 1, wherein the processor is configured for determining whether the candidate activity level was at steady state by determining whether the bin value for the candidate activity level is within a first range of a bin value for a previous activity level, determining whether the bin value for the candidate activity level is within a second range of bin values for a sequence of previous activity levels, the second range larger than the first range, and determining whether activity levels are trending upward or downward using bin values from activity levels detected before and after the candidate activity level.

20. The system of claim 19, wherein the first range is one bin and the second range is two bins.

21. The system of claim 1, further comprising a resulting indicator determined by using the aggregated physiological data averages.

22. The system of claim 21, wherein the resulting indicator is determined using the aggregated physiological data averages to aggregate heart rate, stroke volume, tidal volume, respiratory rate, pulmonary artery diastolic pressure data, or venous blood oxygen level.

23. The system of claim 1, wherein the processor (550) is configured for determining whether the candidate activity level was at steady state using bin values from activity levels detected before and after the candidate activity level by deeming the candidate activity level as not at steady state if activity levels from a specified amount of time preceding the candidate activity level are not within a specified range.

24. A machine-assisted method comprising:
detecting a physical activity signal, using an implantable medical device (500), from a human or animal subject;
simultaneously detecting a different first other physiological signal, from the subject, using the implantable medical device;
processing the physical activity signal to define a first time period associated with a first steady-state physical activity level of the subject, including identifying for the first time period a first beginning time at which the physical activity of the subject is deemed to be at steady state at the first steady-state physical activity level, and a first ending time at which the subject leaves the first steady-state physical activity level;
obtaining a first indicator by combining data from the first other physiological signal obtained during the first time period;
processing the physical activity signal to define a second time period associated with a second steady-state physical activity level of the subject, including identifying for the second time period a second beginning time at which the physical activity of the subject is deemed to be at steady state at the second steady-state physical activity level, and a second ending time at which the subject leaves the second steady-state physical activity level;
obtaining a second indicator by combining data from the first other physiological signal obtained during the second time period; and
using the first and second indicators to provide a resulting indicator,
the physical activity signal falling within an activity range divided into activity bins, wherein
the first steady-state physical activity level corresponds to an activity bin associated with a range of activity levels,
the second steady-state physical activity level of the subject corresponding to the same activity bin as the first steady-state physical activity level of the subject,
and wherein data from the first other physiological signal also obtained during the first and second time periods are combined and
the combined data from the first other physiological signal are used to provide a resulting indicator,
wherein the method also comprises:
determining for a candidate activity level an activity level bin value corresponding to a range of activity level values;
determining whether the candidate activity level was at steady state using bin values from activity levels detected before and after the candidate activity level;
if the candidate activity level was at steady state, adding to one or more sum registers physiological data detected contemporaneously with the candidate activity level using an implantable medical device, and incrementing one or more counters;
if the candidate activity level was not at steady state and a previous activity level was at steady state:
averaging the physiological data using the one or more sum registers and one or more counters;
aggregating the averaged physiological data with previous physiological data detected at an activity level corresponding to the same bin value; and
storing aggregated physiological data averages.

25. The method of claim 24, in which the second steady-state physical activity level corresponds to a different activity bin than the first steady-state physical activity level.

26. The method of claim 24, in which the second steady-state physical activity level corresponds to the same activity bin as the first steady-state physical activity level.

27. The method of claim 26, wherein the second time period occurs after the first time period, and the obtaining a second indicator by combining data from the first other physiological signal obtained during the second time period includes combining data from the first other physiological signal obtained during the second time period with data from the first other physiological signal obtained during the first time period.

28. The method of claim 27, further comprising processing the physical activity signal to define a third time period associated with a third steady-state physical activity level of the subject, including identifying for the third time period a third beginning time at which the physical activity of the subject is deemed to be at steady state at the third steady-state physical activity level, and a third ending time at which the subject leaves the third steady-state physical activity level, the third time period occurring between the first time period and the second time period; and
obtaining a third indicator by combining data from the first other physiological signal obtained during the third time period.

29. The method of claim 24, further comprising detecting a second other physiological signal, from the subject, using the implantable medical device, and obtaining third and fourth indicators by combining data from the second other physiological signal obtained during the respective first and second time periods, wherein using the first and second indicators to provide a resulting indicator includes using the first, second, third and fourth indicators.

30. The method of claim 24, wherein obtaining a first indicator by combining data from the first other physiological signal obtained during the first time period includes averaging the data from the first other physiological signal obtained during the first time period.

31. The method of claim 24, wherein the resulting indicator includes an offset, a difference, a derivative, an interpolation, or an integral.

32. The method of claim 24, wherein the resulting indicator provides information about a relationship between heart rate and activity.

33. The method of claim 24, wherein the resulting indicator provides information about a relationship between stroke volume and activity or heart rate.

34. The method of claim 24, wherein the resulting indicator provides information about a relationship between at least two of respiratory rate, activity, and tidal volume.

35. The method of claim 24, wherein the resulting indicator provides information about a relationship between an intravascular pressure or venous blood oxygen saturation and activity level or one or more other physiological parameters.

36. The method of claim 24, further comprising:
processing the physical activity signal to define a third time period associated with a third steady-state physical activity level of the subject, including identifying for the third time period a third beginning time at which the physical activity of the subject is deemed to be at steady state at the third steady-state physical activity level, and a third ending time at which the subject leaves the third steady-state physical activity level, the third steady-state physical activity level corresponding to an activity bin;
processing the physical activity signal to define a fourth time period associated with a fourth steady-state physical activity level of the subject, the fourth steady-state physical activity level of the subject corresponding to the same activity bin as the first steady-state physical activity level of the subject, including identifying for the fourth time period a fourth beginning time at which the physical activity of the subject is deemed to be at steady state at the fourth steady-state physical activity level, and a fourth ending time at which the subject leaves the fourth steady-state physical activity level;
combining data from the first other physiological signal also obtained during the third and fourth time periods;
using the combined data from the first other physiological signal obtained during the third and fourth time periods to provide a second resulting indicator; and
using the resulting indicator from the first and second time periods and the resulting indicator from the third and fourth time periods to monitor a physiological condition of the subject.

37. The method of claim 24, wherein combining data from the first other physiological signal also obtained during the first and second time periods includes determining a first average of data obtained during the first time period, obtaining a second average of data obtained during the second time period, and combining the first average and the second average.

38. The method of claim 24, wherein using the combined data from the first other physiological signal to provide a resulting indicator includes determining a heart rate, stroke volume, tidal volume, or respiration rate for a steady state activity level.

39. The method of claim 24, wherein using the combined data from the first other physiological signal to provide a resulting indicator includes determining a venous blood oxygen saturation or pulmonary artery diastolic pressure for a steady state activity level.

40. The method of claim 24, wherein determining whether the candidate activity level was at steady state includes:
determining whether the candidate activity level is within a first range of a previous activity level;
determining whether the candidate activity level is within a second range of a sequence of previous activity levels, the second range larger than the first range; and
determining whether activity levels are trending upward or downward using activity levels detected before and after the candidate activity level.

41. The method of claim 24, wherein determining whether the candidate activity level was at steady state includes:
determining whether the bin value for the candidate activity level is within a first range of a bin value for a previous activity level;
determining whether the bin value for the candidate activity level is within a second range of bin values for a sequence of previous activity levels, the second range larger than the first range; and
determining whether activity levels are trending upward or downward using bin values from activity levels detected before and after the candidate activity level.

42. The method of claim 41, wherein the first range is one bin and the second range is two bins.

43. The method of claim 24, further comprising using the aggregated physiological data averages to provide a resulting indicator.

44. The method of claim 43, wherein using the aggregated physiological data averages to provide a resulting indicator includes aggregating heart rate, stroke volume, tidal volume, respiratory rate, pulmonary artery diastolic pressure data, or venous blood oxygen level.

45. The method of claim 24, wherein determining whether the candidate activity level was at steady state using bin values from activity levels detected before and after the candidate activity level includes deeming the candidate activity level as not at steady state if activity levels from a specified amount of time preceding the candidate activity level are not within a specified range.

46. The system of claim 1, with an implantable second other physiological signal sensor, configured for detecting a second other physiological signal from the subject, and obtaining third and fourth indicators by using data from the second other physiological signal obtained during the respective first and second time periods, wherein using the first and second indicators to provide the resulting indicator includes using the first, second, third, and fourth indicators to provide the resulting indicator, wherein the resulting indicator represents an upward or downward shift in the relationship between the first other physiological signal and a second other physiological signal.

47. The system of claim 1 or 46, wherein the first physiological signal is indicative of at least one of the following: heart rate, cardiac output, stroke volume, respiratory rate, tidal volume, minute ventilation, an intravascular pressure, or venous blood oxygen saturation.

48. The system of claim 46 or 47, wherein the second physiological signal is indicative of at least one of the following: heart rate, cardiac output, stroke volume, respiratory rate, tidal volume, minute ventilation, an intravascular pressure, or venous blood oxygen saturation.

49. The method of claim 24 comprising detecting a second other physiological signal, from the subject, using the implantable medical device, and obtaining third and fourth indicators by combining data from the second other physiological signal obtained during the respective first and second time periods, wherein using the first and second indicators to provide a resulting indicator includes using the first, second, third and fourth indicators, wherein the resulting indicator represents an upward or downward shift in the relationship between the first other physiological signal and a second other physiological signal.

50. The method of claim 24 or 49, wherein the first physiological signal is indicative of at least one of the following: heart rate, cardiac output, stroke volume, respiratory rate, tidal volume, minute ventilation, an intravascular pressure, or venous blood oxygen saturation.

51. The method of claim 49 or 50, wherein the second physiological signal is indicative of at least one of the following: heart rate, cardiac output, stroke volume, respiratory rate, tidal volume, minute ventilation, and intravascular pressure, or venous blood oxygen saturation.

## Patentansprüche

1. System, welches aufweist:
einen implantierbaren Aktivitätssensor (555), der konfiguriert ist zum Erfassen eines Körperaktivitätssignals eines menschlichen oder tierischen Lebewesens;
einen implantierbaren ersten anderen physiologischen Sensor (505, 510, 515, 525, 560, 565), der konfiguriert ist zum Erfassen eines ersten anderen physiologischen Signals von dem Lebewesen, wobei das erste andere physiologische Signal verschieden von dem Körperaktivitätssignal ist;
einen Signalprozessor (550), der konfiguriert ist zum Verarbeiten des Körperaktivitätssignals, um eine erste Zeitperiode, die mit einem ersten Steady-State-Pegel der Körperaktivität des Lebewesens assoziiert ist, zu definieren, enthaltend für die erste Zeitperiode das Identifizieren einer ersten Anfangszeit, zu der die körperliche Aktivität des Lebewesens als in einem Steady State auf dem ersten Steady-State-Pegel der körperlichen Aktivität erachtet wird, und einer ersten Beendigungszeit, zu der das Lebewesen den ersten Steady-State-Pegel der körperlichen Aktivität verlässt, wobei der Signalprozessor einen ersten Indikator erhält durch Verwendung von Daten von dem ersten anderen physiologischen Signal, das während der ersten Zeitperiode erhalten wurde, der Signalprozessor das Körperaktivitätssignal verarbeitet, um eine zweite Zeitperiode zu definieren, die mit einem zweiten Steady-State-Pegel der körperlichen Aktivität des Lebewesens assoziiert ist, enthaltend für die zweite Zeitperiode das Identifizieren einer zweiten Anfangszeit, zu der die körperliche Aktivität des Lebewesens als in einem Steady State auf dem zweiten Steady-State-Pegel der körperlichen Aktivität erachtet wird, und einer zweiten Beendigungszeit, zu der das Lebewesen den zweiten Steady-State-Pegel der körperlichen Aktivität verlässt, wobei der Signalprozessor einen zweiten Indikator durch Verwendung von Daten von dem ersten anderen physiologischen Signal, das während der zweiten Zeitperiode erhalten wurde, erhält, und der Signalprozessor den ersten und den zweiten Indikator verwendet, um einen ersten resultierenden Indikator bereitzustellen,
wobei der Prozessor (550) konfiguriert ist zum Definieren eines ersten Aktivitätsfachs für einen Bereich von Aktivitätspegeln, die den ersten Steady-State-Pegel der körperlichen Aktivität umfassen, und wobei das System weiterhin einen implantierbaren Beschleunigungsmesser (555) aufweist, der konfiguriert ist zum Erfassen mehrerer Aktivitätspegel, der Prozessor (550) Aktivitätspegel-Fachzähler und Summenregister für physiologische Daten enthält, der Prozessor konfiguriert ist zum Bestimmen eines Aktivitätspegel-Fachwerts entsprechend einem Bereich von Aktivitätspegelwerten für einen Kandidatenaktivitätspegel und zum Bestimmen, ob der Kandidatenaktivitätspegel in einem Steady State war, unter Verwendung von Fachwerten von Aktivitätspegeln, die vor und nach dem Kandidatenaktivitätspegel erfasst wurden, und, wenn bestimmt wird, dass der Kandidatenaktivitätspegel in einem Steady State ist, dann Hinzufügen von physiologischen Daten, die gleichzeitig mit dem Kandidatenaktivitätspegel erfasst wurden, zu einem oder mehreren der Summenregister, und Inkrementieren von einem oder mehreren der Zähler, und wenn bestimmt wird, dass der Aktivitätspegel nicht in einem Steady State ist und ein vorhergehender Aktivitätspegel in einem Steady State war, dann Bilden des Durchschnitts der physiologischen Daten unter Verwendung des einen oder der mehreren Summenregister und des einen oder der mehreren der Zähler, und wobei der Prozessor weiterhin konfiguriert ist zum Vereinigen des Durchschnitts der physiologischen Daten mit vorhergehenden physiologischen Daten, die bei einem Aktivitätspegel entsprechend demselben Fachwert erfasst wurden, und wobei der Prozessor einen Speicher zum Speichern der zusammengefassten Durchschnittswerte der physiologischen Daten enthält oder mit diesem gekoppelt ist.

2. System nach Anspruch 1, bei dem der Prozessor (550) ein zweites Aktivitätsfach für einen Bereich von Aktivitätspegeln, die den zweiten Steady-State-Körperaktivitätspegel umfassen, enthält, wobei das erste und das zweite Aktivitätsfach verschiedenen Körperaktivitätspegeln entsprechen.

3. System nach Anspruch 1, bei dem der zweite Steady-State-Körperaktivitätspegel demselben ersten Aktivitätsfach wie der erste Steady-State-Körperaktivitätspegel entspricht.

4. System nach Anspruch 3, bei dem die zweite Zeitperiode nach der ersten Zeitperiode auftritt und der zweite Indikator Daten von dem ersten anderen physiologischen Signal, das während der zweiten Zeitperiode erhalten wurde, mit Daten von dem ersten anderen physiologischen Signal, das während der ersten Zeitperiode erhalten wurde, kombiniert.

5. System nach Anspruch 4, bei dem der Prozessor (550) konfiguriert ist zum Verarbeiten des Körperaktivitätssignals zum Definieren einer dritten Zeitperiode, die mit einem dritten Steady-State-Körperaktivitätspegel des Lebewesens assoziiert ist, enthaltend für die dritte Zeitperiode das Identifizieren einer dritten Anfangszeit, zu der die körperliche Aktivität des Lebewesens als in einem Steady State bei dem dritten Steady-State-Pegel der körperlichen Aktivität erachtet wird, und einer dritten Beendigungszeit, zu der das Lebewesen den dritten Steady-State-Pegel der körperlichen Aktivität verlässt, wobei die dritte Zeitperiode zwischen der ersten Zeitperiode und der zweiten Zeitperiode auftritt, und das Erhalten eines dritten Indikators durch Kombinieren von Daten aus dem ersten anderen physiologischen Signal, das während der dritten Zeitperiode erhalten wurde.

6. System nach Anspruch 1, weiterhin aufweisend einen implantierbaren Sensor für ein zweites anderes physiologisches Signal, der konfiguriert ist zum Erfassen eines zweiten anderen physiologischen Signals von dem Lebewesen, und zum Erhalten eines dritten und eines vierten Indikators durch Verwendung von Daten von dem zweiten anderen physiologischen Signal, das während der jeweiligen ersten und zweiten Zeitperiode erhalten wurde, wobei die Verwendung des ersten und des zweiten Indikators zum Bereitstellen des resultierenden Indikators die Verwendung des ersten, zweiten, dritten und vierten Indikators zum Bereitstellen des resultierenden Indikators enthält.

7. System nach Anspruch 1, bei dem der Prozessor (550) konfiguriert zum Erhalten des ersten Indikators durch Verwendung von Daten aus dem ersten anderen physiologischen Signal, das während der ersten Zeitperiode erhalten wurde durch Bildung des Durchschnitts der Daten aus dem ersten anderen physiologischen Signal, das während der ersten Zeitperiode erhalten wurde,

8. System nach Anspruch 1, bei dem der resultierende Indikator eine Versetzung, eine Differenz, eine Ableitung, eine Interpolation oder ein Integral enthält.

9. System nach Anspruch 1, bei dem der resultierende Indikator Informationen über eine Beziehung zwischen Herzfrequenz und Aktivität bereitstellt.

10. System nach Anspruch 1, bei dem der resultierende Indikator Informationen über eine Beziehung zwischen Herzschlagvolumen und Aktivität oder Herzfrequenz bereitstellt.

11. System nach Anspruch 1, bei dem der resultierende Indikator Informationen über eine Beziehung zwischen zumindest zwei von Atmungsfrequenz, Aktivität und Atemzugvolumen bereitstellt.

12. System nach Anspruch 1, bei dem der resultierende Indikator Informationen über eine Beziehung zwischen einem intravaskulären Druck oder der Sauerstoffsättigung von venösem Blut und dem Aktivitätspegel oder einem oder mehreren anderen physiologischen Parametern bereitstellt.

13. System nach Anspruch 1, welches aufweist:
einen implantierbaren Aktivitätssensor (555), der konfiguriert ist zum Erfassen eines Körperaktivitätssignals von einem menschlichen oder tierischen Lebewesen, wobei das Körperaktivitätssignal in einen Aktivitätsbereich, der in Aktivitätsfächer unterteilt ist, fällt;
einen implantierbaren ersten anderen physiologischen Sensor (505, 510, 515, 525, 560, 565), der konfiguriert ist zum gleichzeitigen Erfassen eines verschiedenen ersten anderen physiologischen Signals von dem Lebewesen;
einen Prozessor (550), der konfiguriert ist zum Verarbeiten des Körperaktivitätssignals, um eine erste Zeitperiode, die mit einem ersten Steady-State-Pegel der körperlichen Aktivität des Lebewesens assoziiert ist, zu definieren, enthaltend für die erste Zeitperiode das Identifizieren einer ersten Anfangszeit, zu der die körperliche Aktivität des Lebewesens als in einem Steady State bei dem ersten Steady-State-Pegel der körperlichen Aktivität erachtet wird, und einer ersten Beendigungszeit, zu der das Lebewesen den ersten Steady-State-Pegel der körperlichen Aktivität verlässt, wobei der erste Steady-State-Pegel der körperlichen Aktivität einem Aktivitätsfach entspricht, der Prozessor (550) konfiguriert ist zum Verarbeiten des Körperaktivitätssignals, um eine zweite Zeitperiode zu definieren, die mit einem zweiten Steady-State-Pegel der körperlichen Aktivität des Lebewesens assoziiert ist, wobei der zweite Steady-State-Pegel der körperlichen Aktivität des Lebewesense demselben Aktivitätsfach wie der erste Steady-State-Pegel der körperlichen Aktivität des Lebewesens entspricht, enthaltend für die zweite Zeitperiode das Identifizieren einer zweiten Anfangszeit, zu der die körperliche Aktivität des Lebewesens als in einem Steady State auf dem zweiten Steady-State-Pegel der körperlichen Aktivität erachtet wird, und einer zweiten Beendigungszeit, zu der das Lebewesen den zweiten Steady-State-Pegel der körperlichen Aktivität verlässt, und der Prozessor Daten aus dem ersten anderen physiologischen Signal, das auch während der ersten und der zweiten Zeitperiode erhalten wurde, kombiniert, und der Prozessor einen resultierenden Indikator unter Verwendung der kombinierten Daten aus dem ersten anderen physiologischen Signal bestimmt.

14. System nach Anspruch 13, bei dem der Prozessor (550) konfiguriert ist zum Verarbeiten des Körperaktivitätssignals, um eine dritte Zeitperiode, die mit einem dritten Steady-State-Pegel der körperlichen Aktivität des Lebewesens assoziiert ist, zu definieren, enthaltend für die dritte Zeitperiode das Identifizieren einer dritten Anfangszeit, zu der die körperliche Aktivität des Lebewesens als in einem Steady State auf dem dritten Steady-State-Pegel der körperlichen Aktivität erachtet wird, und einer dritten Beendigungszeit, zu der das Lebewesen den dritten Steady-State-Pegel der körperlichen Aktivität verlässt, wobei der dritte Steady-State-Pegel der körperlichen Aktivität einem Aktivitätsfach entspricht, und wobei der Prozessor konfiguriert ist zum Verarbeiten des Körperaktivitätssignals, um eine vierte Zeitperiode, die mit einem vierten Steady-State-Pegel der körperlichen Aktivität des Lebewesens assoziiert ist, zu definieren, wobei der vierte Steady-State-Pegel der körperlichen Aktivität des Lebewesens demselben Aktivitätsfach wie der erste Steady-State-Pegel der körperlichen Aktivität des Lebewesens entspricht, enthaltend für die vierte Zeitperiode das Identifizieren einer vierten Anfangszeit, zu der die körperliche Aktivität des Lebewesens als in einem Steady State auf dem vierten Steady-State-Pegel der körperlichen Aktivität erachtet wird, und einer vierten Beendigungszeit, zu der das Lebewesen den vierten Steady-State-Pegel der körperlichen Aktivität verlässt, und wobei der Prozessor konfiguriert ist zum Kombinieren von Daten aus dem anderen physiologischen Signal, das auch während der dritten und der vierten Zeitperiode erhalten wurde, und wobei der Prozessor einen zweiten resultierenden Indikator bestimmt durch Verwenden der kombinierten Daten aus dem ersten anderen physiologischen Signal, das während der dritten und der vierten Zeitperiode erhalten wurde, und wobei der Prozessor konfiguriert ist zum Verwenden des ersten und des zweiten resultierenden Indikators, um einen physiologischen Zustand des Lebewesens zu überwachen.

15. System nach Anspruch 13, bei dem der Prozessor (550) konfiguriert ist zum Kombinieren von Daten aus dem ersten anderen physiologischen Signal, das während der ersten und der zweiten Zeitperiode erhalten wurde durch Bestimmen eines ersten Durchschnitts von Daten, die während der ersten Zeitperiode erhalten wurden, Erhalten eines zweiten Durchschnitts von Daten, die während der zweiten Zeitperiode erhalten wurden, und Kombinieren des ersten Durchschnitts und des zweiten Durchschnitts.

16. System nach Anspruch 13, bei dem der Prozessor (550) konfiguriert ist zum Bestimmen des ersten resultierenden indikators unter Verwendung der kombinierten Daten aus dem ersten anderen physiologischen Signal durch Bestimmen einer Herzfrequenz, eines Herzschlagvolumens, eines Atemzugvolumens oder einer Atmungsfrequenz für einen Steady-State-Aktivitätspegel.

17. System nach Anspruch 13, bei dem der Prozessor (550) konfiguriert ist zum Bestimmen des ersten resultierenden Indikators unter Verwendung der kombinierten Daten aus dem ersten anderen physiologischen Signal durch Bestimmen einer Sauerstoffsättigung von venösem Blut oder eines diastolischen Drucks der Lungenarterie für einen Steady-State-Aktivitätspegel.

18. System nach Anspruch 1, bei dem der Prozessor (550) konfiguriert ist zum Bestimmen, ob der Aktivitätspegelkandidat in einem Steady State war, durch Bestimmen, ob der Aktivitätspegelkandidat innerhalb eines ersten Bereichs eines früheren Aktivitätspegels ist, Bestimmen, ob der Aktivitätspegelkandidat innerhalb eines zweiten Bereichs einer Folge von früheren Aktivitätspegeln ist, wobei der zweite Bereich größer als der erste Bereich ist, und Bestimmen, ob die Aktivitätspegel einen Aufwärts- oder Abwärtstrend haben, unter Verwendung der Aktivitätspegel, die vor und nach dem Aktivitätspegelkandidaten erfasst wurden.

19. System nach Anspruch 1, bei dem der Prozessor konfiguriert ist zum Bestimmen, ob der Aktivitätspegelkandidat im Steady State war, durch Bestimmen, ob der Fachwert für den Aktivitätspegelkandidaten innerhalb eines ersten Bereichs eines Fachwerts für einen vorhergehenden Aktivitätspegel ist, Bestimmen, ob der Fachwert für den Alctivitätspegelkandidaten innerhalb eines zweiten Bereichs von Fachwerten für eine Folge von vorhergehenden Aktivitätspegeln ist, wobei der zweite Bereich größer als der erste Bereich ist, und Bestimmen, ob Aktivitätspegel einen Aufwärts- oder Abwärtstrend haben, unter Verwendung von Fachwerten von Aktivitätspegeln, die vor und nach dem Aktivitätspegelkandidaten erfasst wurden.

20. System nach Anspruch 19, bei dem der erste Bereich ein Fach ist und der zweite Bereich zwei Fächer sind,

21. System nach Anspruch 1, weiterhin aufweisend einen resultierenden Indikator, der durch Verwendung der zusammengefassten Durchschnittswerte der physiologischen Daten bestimmt wurde.

22. System nach Anspruch 21, bei dem der resultierende Indikator durch Verwendung der zusammengefassten Durchschnitte physiologischer Daten zum Zusammenfassen von Herzfrequenz, Herzschlagvolumen, Atemzugvolumen, Atmungsfrequenz, diastolischer Druckdaten der Lungenarterie oder des Sauerstoffpegels von venösem Blut bestimmt wird,

23. System nach Anspruch 1, bei dem der Prozessor (550) konfiguriert ist zum Bestimmen, ob der Aktivitätspegelkandidat im Steady State war, unter Verwendung von Fachwerten von Aktivitätspegeln, die vor und nach dem Aktivitätspegelkandidaten erfasst wurden, durch Erachten des Aktivitätspegelkandidaten als nicht im Steady State , wenn Aktivitätspegel aus einer bestimmten Zeitspanne, die dem Aktivitätspegelkandidaten vorhergeht, nicht innerhalb eines bestimmten Bereichs sind.

24. Maschinenunterstütztes Verfahren, welches aufweist:
Erfassen eines Körperaktivitätssignals eines menschlichen oder tierischen Lebewesens unter Verwendung einer implantierbaren medizinischen Vorrichtung (500);
gleichzeitiges Erfassen eines verschiedenen ersten anderen physiologischen Signals von dem Lebewesen unter Verwendung einer implantierbaren medizinischen Vorrichtung;
Verarbeiten des Körperaktivitätssignals, um eine erste Zeitperiode, die mit einem ersten Steady-State-Pegel der körperlichen Aktivität des Lebewesens assoziiert ist, zu definieren, enthaltend für die erste Zeitperiode das Identifizieren einer ersten Anfangszeit, zu der die körperliche Aktivität des Lebewesens als in einem Steady State auf dem ersten Steady-State-Pegel der körperlichen Aktivität erachtet wird, und einer ersten Beendigungszeit, zu der das Lebewesen den ersten Steady-State-Pegel der körperlichen Aktivität verlässt;
Erhalten eines ersten Indikators durch Kombinieren von Daten aus dem ersten anderen physiologischen Signal, das während der ersten Zeitperiode erhalten wurde;
Verarbeiten des Körperaktivitätssignals zum Definieren einer zweiten Zeitperiode, die mit einem zweiten Steady-State-Pegel der körperlichen Aktivität des Lebewesens assoziiert ist, enthaltend für die zweite Zeitperiode das Identifizieren einer zweiten Anfangszeit, zu der die körperliche Aktivität des Lebewesens als in einem Steady State auf dem zweiten Steady-State-Pegel der körperlichen Aktivität erachtet wird, und einer zweiten Beendigungszeit, zu der das Lebewesen den zweiten Steady-State-Pegel der körperlichen Aktivität verlässt;
Erhalten eines zweiten Indikators durch Kombinieren von Daten aus dem ersten anderen physiologischen Signal, das während der zweiten Zeitperiode erhalten wurde; und
Verwenden des ersten und des zweiten Indikators, um einen resultierenden Indikator bereitzustellen,
wobei das Körperaktivitätssignal in einen Aktivitätsbereich fällt, der in Aktivitätsfächer unterteilt ist, wobei
der erste Steady-State-Pegel der körperlichen Aktivität einem Aktivitätsfach entspricht, das mit einem Bereich von Aktivitätspegeln assoziiert ist,
der zweite Steady-State-Pegel der körperlichen Aktivität des Lebewesens demselben Aktivitätsfach wie der erste Steady-State-Pegel der körperlichen Aktivität des Lebewesens entspricht,
und wobei Daten aus dem ersten anderen physiologischen Signal, das auch während der ersten und der zweiten Zeitperiode erhalten wurde, kombiniert werden und
die kombinierten Daten aus dem ersten anderen physiologischen Signal verwendet werden, um einen resultierenden Indikators bereitzustellen,
wobei das Verfahren auch aufweist:
Bestimmen eines Aktivitätspegel-Fachwerts für einen Aktivitätspegelkandidaten entsprechend einem Bereich von Aktivitätspegelwerten;
Bestimmen, ob der Aktivitätspegelkandidat in einem Steady State war, unter Verwendung von Fachwerten von Aktivitätspegeln, die vor und nach dem Aktivitätspegelkandidaten erfasst wurden;
wenn der Aktivitätspegelkandidat in einem Steady State war, Hinzufügen von physiologischen Daten zu einem oder mehreren Summenregistern, die gleichzeitig mit dem Aktivitätspegelkandidaten unter Verwendung einer implantierbaren medizinischen Vorrichtung erfasst wurden, und Inkrementieren eines oder mehrerer Zähler;
wenn der Aktivitätspegelkandidat nicht einem Steady State war und ein vorhergehender Aktivitätspegel in einem Steady State war:
Bilden des Durchschnitts der physiologischen Daten unter Verwendung des einen oder der mehreren Summenregister und eines oder mehrerer Zähler;
Zusammenfassen des Durchschnitts der physiologischen Daten mit vorhergehenden physiologischen Daten, die bei einem Aktivitätspegel, der demselben Fachwert entspricht, erfasst wurden; und
Speichern der zusammengefassten Durchschnitte der physiologischen Daten.

25. Verfahren nach Anspruch 24, bei dem der zweite Steady-State-Pegel der körperlichen Aktivität einem anderen Aktivitätsfach als der erste Steady-State-Pegel der körperlichen Aktivität entspricht.

26. Verfahren nach Anspruch 24, bei dem der zweite Steady-State-Pegel der körperlichen Aktivität demselben Aktivitätsfach wie der erste Steady-State-Pegel der körperlichen Aktivität entspricht.

27. Verfahren nach Anspruch 26, bei dem die zweite Zeitperiode nach der ersten Zeitperiode auftritt und das Erhalten eines zweiten Indikators durch Kombinieren von Daten von dem ersten anderen physiologischen Signal, das während der zweiten Zeitperiode erhalten wurde, das Kombinieren von Daten aus dem ersten anderen physiologischen Signal, das während der zweiten Zeitperiode erhalten wurde, mit Daten aus dem ersten anderen physiologischen Signal, das während der ersten Zeitperiode erhalten wurde, enthält,

28. Verfahren nach Anspruch 27, weiterhin aufweisend das Verarbeiten des Körperaktivitätssignals, um eine dritte Zeitperiode, die mit dem dritten Steady-State-Pegel der körperlichen Aktivität des Lebewesens assoziiert ist, zu definieren, enthaltend für die dritte Zeitperiode das Identifizieren einer dritten Anfangszeit, zu der die körperliche Aktivität des Lebewesens als ein Steady State auf dem dritten Steady-State-Pegel der körperlichen Aktivität erachtet wird, und einer dritten Beendigungszeit, zu der das Lebewesen den dritten Steady-State-Pegel der körperlichen Aktivität verlässt, wobei die dritte Zeitperiode zwischen der ersten Zeitperiode und der zweiten Zeitperiode auftritt; und
Erhalten eines dritten Indikators durch Kombinieren von Daten aus dem ersten anderen physiologischen Signal, das während der dritten Zeitperiode erhalten wurde.

29. Verfahren nach Anspruch 24, weiterhin aufweisend das Erfassen eines zweiten anderen physiologischen Signals von dem Lebewesen unter Verwendung der implantierbaren medizinischen Vorrichtung und das Erhalten eines dritten und eines vierten Indikators durch Kombinieren von Daten aus dem zweiten anderen physiologischen Signal, das während der jeweiligen ersten und zweiten Zeitperiode erhalten wurde, wobei die Verwendung des ersten und des zweiten indikators zum Bereitstellen eines resultierenden Indikators die Verwendung des ersten, zweiten, dritten und vierten Indikators enthält.

30. Verfahren nach Anspruch 24, bei dem das Erhalten eines ersten Indikators durch Kombinieren von Daten aus dem ersten anderen physiologischen Signal, das während der ersten Zeitperiode erhalten wurde, die Bildung des Durchschnitts der Daten aus dem ersten anderen physiologischen Signal, das während der ersten Zeitperiode erhalten wurde, enthält.

31. Verfahren nach Anspruch 24, bei dem der resultierende Indikator eine Versetzung, eine Differenz, eine Ableitung, eine Interpolation oder ein Integral enthält.

32. Verfahren nach Anspruch 24, bei dem der resultierende Indikator Informationen über eine Beziehung zwischend der Herzfrequenz und der Aktivität bereitstellt.

33. Verfahren nach Anspruch 24, bei dem der resultierende Indikator Informationen über eine Beziehung zwischen dem Herzschlagvolumen und der Aktivität oder Herzfrequenz bereitstellt.

34. Verfahren nach Anspruch 24, bei dem der resultierende Indikator Informationen über eine Beziehung zwischen zumindest zwei von der Atmungsfrequenz, der Aktivität und dem Atemzugvolumen bereitstellt.

35. Verfahren nach Anspruch 24, bei dem der resultierende Indikator Informationen über eine Beziehung zwischen einem intravaskulären Druck oder der Sauerstoffsättigung von venösem Blut und dem Aktivitätspegel oder einem oder mehreren anderen physiologischen Parametern bereitstellt.

36. Verfahren nach Anspruch 24, weiterhin aufweisend:
Verarbeiten des Körperaktivitätssignals, um eine dritte Zeitperiode, die mit einem dritten Steady-State-Pegel der körperlichen Aktivität des Lebewesens assoziiert ist, zu definieren, enthaltend für die dritte Zeitperiode das Identifizieren einer dritten Anfangszeit, zu der die körperliche Aktivität des Lebewesens als ein Steady State auf dem dritten Steady-State-Pegel der körperlichen Aktivität erachtet wird, und einer dritten Beendigungszeit, zu der das Lebewesen den dritten Steady-State-Pegel der körperlichen Aktivität verlässt, wobei der dritte Steady-State-Pegel der körperlichen Aktivität einem Aktivitätsfach entspricht;
Verarbeiten des Körperaktivitätssignals, um eine vierte Zeitperiode, die mit einem vierten Steady-State-Pegel der körperlichen Aktivität des Lebewesens assoziiert ist, zu definieren, wobei der vierte Steady-State-Pegel der körperlichen Aktivität des Lebewesens demselben Aktivitätsfach wie der erste Steady-State-Pegel der körperlichen Aktivität des Lebewesens entspricht, enthaltend für die vierte Zeitperiode das Identifizieren einer vierten Anfangszeit, zu der die körperliche Aktivität des Lebewesens als in einem Steady State auf dem vierten Steady-State-Pegel der körperlichen Aktivität erachtet wird, und einer vierten Beendigungszeit, zu der das Lebewesen den vierten Steady-State-Pegel der körperlichen Aktivität verlässt;
Kombinieren von Daten aus dem ersten anderen physiologischen Signal, das auch während der dritten und der vierten Zeitperiode erhalten wurde;
Verwenden der kombinierten Daten aus dem anderen physiologischen Signal, das während der ersten und der vierten Zeitperiode erhalten wurde, um einen zweiten resultierenden Indikator bereitzustellen; und
Verwenden des resultierenden Indikator aus der ersten und der zweiten Zeitperiode und des resultierenden Indikators aus der dritten und der vierten Zeitperiode, um einen physiologischen Zustand des Lebewesens zu überwachen.

37. Verfahren nach Anspruch 24, bei dem das Kombinieren von Daten aus dem ersten physiologischen Signal, das auch während der ersten und der zweiten Zeitperiode erhalten wurde, das Bestimmen eines ersten Durchschnitts von Daten, die während der ersten Zeitperiode erhalten wurden, das Erhalten eines zweiten Durchschnitts von Daten, die während der zweiten Zeitperiode erhalten wurden, und das Kombinieren des ersten Durchschnitts und des zweiten Durchschnitts enthält.

38. Verfahren nach Anspruch 24, bei dem das Verwenden der kombinierten Daten aus dem ersten anderen physiologischen Signal zum Bereitstellen eines resultierenden Indikators das Bestimmen einer Herzfrequenz, eines Herzschlagvolumens, eines Atemzugvolumens oder einer Atmungsfrequenz für einen Steady-State-Aktivitätspegel enthält.

39. Verfahren nach Anspruch 24, bei dem das Verwenden der kombinierten Daten aus dem ersten anderen physiologischen Signal zum Bereitstellen eines resultierenden Indikators das Bestimmen einer Sauerstoffsättigung von venösem Blut oder eines diastolischen Drucks der Lungenarterie für einen Steady-State-Aktivitätspegel enthält,

40. Verfahren nach Anspruch 24, bei dem das Bestimmen, ob der Aktivitätspegelkandidat in einem Steady State war, enthält:
Bestimmen, ob der Aktivitätspegelkandidat innerhalb eines ersten Bereichs eines vorhergehenden Aktivitätspegels ist;
Bestimmen, ob der Aktivitätspegelkandidat innerhalb eines zweiten Bereichs einer Folge von vorhergehenden Aktivitätspegel ist, wobei der zweite Bereich größer als der erste Bereich ist; und
Bestimmen, ob Aktivitätspegel einen Aufwärts- oder Abwärtstrend haben, unter Verwendung von vor und nach dem Aktivitätspegelkandidaten erfassten Aktivitätspegeln.

41. Verfahren nach Anspruch 24, bei dem das Bestimmen, ob der Aktivitätspegelkandidat in einem Steady State war, enthält:
Bestimmen, ob der Fachwert für den Aktivitätspegelkandidaten innerhalb eines ersten Bereichs eines Fachwerts für einen vorhergehenden Aktivitätspegel ist;
Bestimmen, ob der Fachwert für den Aktivitätspegelkandidaten innerhalb eines zweiten Bereichs von Fachwerten für eine Folge von vorhergehenden Aktivitätspegeln ist, wobei der zweite Bereich größer als der erste Bereich ist; und
Bestimmen, ob Aktivitätspegel einen Aufwärts- oder Abwärtstrend haben, unter Verwendung von Fachwerten von Aktivitätspegeln, die vor und nach dem Aktivitätspegelkandidaten erfasst wurden.

42. Verfahren nach Anspruch 41, bei dem der erste Bereich ein Fach ist und der zweite Bereich zwei Fächer sind.

43. Verfahren nach Anspruch 24, weiterhin aufweisend die Verwendung der zusammengefassten Durchschnitte physiologischer Daten, um einen resultierenden Indikator bereitzustellen.

44. Verfahren nach Anspruch 43, bei dem die Verwendung der vereinigten Durchschnitte physiologischer Daten zum Bereitstellen eines resultierenden Indikators das Zusammenfassen der Herzfrequenz, des Herzschlagvolumens, des Atemzugvolumens, der Atemfrequenz, diastolischer Druckdaten der Lungenarterie oder des Sauerstoffpegels von venösem Blut enthält.

45. Verfahren nach Anspruch 24, bei dem das Bestimmen, ob der Aktivitätspegelkandidat in einem Steady State war, unter Verwendung von Fachwerten von vor und nach dem Aktivitätspegelkandidaten erfassten Aktivitätspegeln das Erachten des Aktivitätspegelkandidaten als nicht im Steady State enthält, wenn Aktivitätspegel aus einer bestimmten Zeitspanne, die dem Aktivitätspegelkandidaten vorhergeht, nicht innerhalb eines bestimmten Bereichs sind.

46. System nach Anspruch 1, mit einem implantierbaren Sensor für ein zweites anderes physiologisches Signal, konfiguriert zum Erfassen eines zweiten anderen physiologischen Signals von dem Lebewesen und zum Erhalten eines dritten und eines vierten Indikators durch Verwendung von Daten aus dem zweiten anderen physiologischen Signal, das während der jeweiligen ersten und zweiten Zeitperiode erhalten wurde, wobei die Verwendung des ersten und des zweiten Indikators zum Bereitstellen des resultierenden Indikators die Verwendung des ersten, zweiten, dritten und vierten Indikators enthält, um den resultierenden Indikator bereitzustellen, wobei der resultierende Indikator eine Aufwärts- oder Abwärtsverschiebung in der Beziehung zwischen dem ersten anderen physiologischen Signal und einem zweiten anderen physiologischen Signal darstellt.

47. System nach Anspruch 1 oder 46, bei dem das erste physiologische Signal einen Hinweis auf zumindest eine/eines/einen der Folgenden bietet: Herzfrequenz, Herzausgangsleistung, Herzschlagvolumen, Atemfrequenz, Atemzugvolumen, Atemminutenvolumen, einen intravaskulären Druck oder Sauerstoffsättigung von venösem Blut.

48. System nach Anspruch 46 oder 47, bei dem das zweite physiologische Signal einen Hinweis für zumindest eine/eines/einen der Folgenden bietet: Herzfrequenz, Herzausgangsleistung, Herzschlagvolumen, Atemfrequenz, Atemzugvolumen, Atemminutenvolumen, einen intravaskulären Druck oder Sauerstoffsättigung von venösem Blut.

49. Verfahren nach Anspruch 24, aufweisend das Erfassen eines zweiten anderen physiologischen Signals von dem Lebewesen unter Verwendung der implantierbaren medizinischen Vorrichtung und das Erhalten eines dritten und eines vierten Indikators durch Kombinieren von Daten aus dem zweiten anderen physiologischen Signal, das während der jeweiligen ersten und zweiten Zeitperiode erhalten wurde, wobei die Verwendung des ersten und des zweiten Indikators zum Bereitstellen eines resultierenden Indikators die Verwendung des ersten, zweiten, dritten und vierten Indikators enthält, wobei der resultierende Indikator eine Aufwärts- oder Abwärtsverschiebung in der Beziehung zwischen dem ersten anderen physiologischen Signal und dem zweiten anderen physiologischen Signal darstellt.

50. Verfahren nach Anspruch 24 oder 49, bei dem das erste physiologische Signal einen Hinweis auf zumindest eine/eines/einen der Folgenden bietet: Herzfrequenz, Herzausgangsleistung, Herzschlagvolumen, Atemfrequenz, Atemzugvolumen, Atemminutenvolumen, einen intravaskulären Druck oder Sauerstoffsättigung von venösem Blut.

51. Verfahren nach Anspruch 49 oder 50, bei dem das zweite physiologische Signal einen Hinweis auf zumindest eine/eines/einen der Folgenden bietet: Herzfrequenz, Herzausgangsleistung, Herzschlagvolumen, Atemfrequenz, Atemzugvolumen, Atemminutenvolumen, einen intravaskulären Druck oder Sauerstoffsättigung von venösem Blut.

## Revendications

1. Système comprenant :
un capteur d'activité implantable (555), qui est configuré de manière à détecter un signal d'activité physique à partir d'un sujet qui est soit un être humain, soit un animal;
un premier autre capteur physiologique implantable (505, 510, 515, 525, 560, 565), qui est configuré de manière à détecter un premier autre signal physiologique à partir du sujet, le premier autre signal physiologique étant différent du signal d'activité physique ;
un processeur de signal (550), qui est configuré de manière à traiter le signal d'activité physique afin de définir une première période temporelle qui est associée à un premier niveau d'activité physique stationnaire du sujet, incluant l'identification, au sein de la première période temporelle, d'un premier temps de début auquel l'activité physique du sujet est considérée comme étant stationnaire au premier niveau d'activité physique stationnaire, et d'un premier temps de fin auquel le sujet quitte le premier niveau d'activité physique stationnaire, le processeur de signal obtenant un premier indicateur en utilisant des données qui proviennent du premier autre signal physiologique qui est obtenu pendant la première période temporelle, le processeur de signal traitant le signal d'activité physique afin de définir une deuxième période temporelle qui est associée à un deuxième niveau d'activité physique stationnaire du sujet, incluant l'identification, au sein de la deuxième période temporelle, d'un deuxième temps de début auquel l'activité physique du sujet est considérée comme étant stationnaire au deuxième niveau d'activité physique stationnaire, et d'un deuxième temps de fin auquel le sujet quitte le deuxième niveau d'activité physique stationnaire, le processeur de signal obtenant un deuxième indicateur en utilisant des données qui proviennent du premier autre signal physiologique qui est obtenu pendant la deuxième période temporelle, le processeur de signal utilisant les premier et deuxième indicateurs afin de fournir un premier indicateur résultant, dans lequel :
le processeur (550) est en outre configuré de manière à définir une première classe de niveaux d'activité pour une plage de niveaux d'activité qui comprend le premier niveau d'activité physique stationnaire, et dans lequel le système comprend en outre :
un accéléromètre implantable (555), qui est configuré de manière à détecter une pluralité de niveaux d'activité, le processeur (550) incluant des compteurs de classes de niveaux d'activité et des registres de sommes de données physiologiques, le processeur étant configuré de manière à déterminer, pour un niveau d'activité candidat, une valeur de classe de niveaux d'activité qui correspond à une plage de valeurs de niveau d'activité, et de manière à déterminer si oui ou non le niveau d'activité candidat était stationnaire en utilisant des valeurs de classe à partir de niveaux d'activité détectés avant et après le niveau d'activité candidat et, lorsque le niveau d'activité candidat est déterminé comme étant stationnaire, pour ajouter alors, à un ou plusieurs des registres de sommes, des données physiologiques détectées de façon contemporaine par rapport au niveau d'activité candidat, et pour incrémenter un ou plusieurs des compteurs, et lorsque le niveau d'activité est déterminé comme n'étant pas stationnaire et qu'un niveau d'activité précédent était stationnaire, pour calculer alors la moyenne des données physiologiques en utilisant les un ou plusieurs registre(s) de sommes et les un ou plusieurs compteur(s), et dans lequel le processeur est en outre configuré de manière à agréger les données physiologiques moyennées avec des données physiologiques précédentes détectées à un niveau d'activité qui correspond à la même valeur de classe, et dans lequel le processeur inclut ou est couplé à une mémoire pour stocker des moyennes de données physiologiques agrégées.

2. Système selon la revendication 1, dans lequel le processeur (550) inclut une seconde classe de niveaux d'activité pour une plage de niveaux d'activité qui comprend le deuxième niveau d'activité physique stationnaire, dans lequel les première et seconde classes de niveaux d'activité correspondent à des niveaux d'activité physique différents,

3. Système selon la revendication 1, dans lequel le deuxième niveau d'activité physique stationnaire correspond à la même première classe de niveaux d'activité que le premier niveau d'activité physique stationnaire.

4. Système selon la revendication 3, dans lequel la deuxième période temporelle survient après la première période temporelle, et le deuxième indicateur combine des données qui proviennent du premier autre signal physiologique obtenu pendant la deuxième période temporelle avec des données qui proviennent du premier autre signal physiologique obtenu pendant la première période temporelle.

5. Système selon la revendication 4, dans lequel le processeur (550) est configuré de manière à traiter le signal d'activité physique afin de définir une troisième période temporelle qui est associée à un troisième niveau d'activité physique stationnaire du sujet, incluant l'identification, au sein de la troisième période temporelle, d'un troisième temps de début auquel l'activité physique du sujet est considérée comme étant stationnaire au troisième niveau d'activité physique stationnaire, et d'un troisième temps de fin auquel le sujet quitte le troisième niveau d'activité physique stationnaire, la troisième période temporelle survenant entre la première période temporelle et la deuxième période temporelle, et l'obtention d'un troisième indicateur en combinant des données qui proviennent du premier autre signal physiologique qui est obtenu prenant la troisième période temporelle.

6. Système selon la revendication 1, comprenant en outre un second autre capteur de signal physiologique implantable, qui est configuré de manière à détecter un second autre signal physiologique à partir du sujet, et de manière à obtenir des troisième et quatrième indicateurs en utilisant des données qui proviennent du second autre signal physiologique qui est obtenu pendant les première et deuxième périodes temporelles respectives, dans lequel l'utilisation des premier et deuxième indicateurs afin de fournir l'indicateur résultant inclut l'utilisation des premier, deuxième, troisième et quatrième indicateurs afin de fournir l'indicateur résultant.

7. Système selon la revendication 1, dans lequel le processeur (550) est configuré de manière à obtenir le premier indicateur en utilisant des données qui proviennent du premier autre signal physiologique qui est obtenu pendant la première période temporelle en calculant la moyenne des données qui proviennent du premier autre signal physiologique qui est obtenu pendant la première période temporelle.

8. Système selon la revendication 1, dans lequel l'indicateur résultant inclut un décalage, une différence, une dérivée, une interpolation ou une intégrale.

9. Système selon la revendication 1, dans lequel l'indicateur résultant fournit une information concernant une relation entre le rythme cardiaque et l'activité,

10. Système selon la revendication 1, dans lequel l'indicateur résultant fournit une information concernant une relation entre le volume systolique et l'activité ou le rythme cardiaque.

11. Système selon la revendication 1, dans lequel l'indicateur résultant fournit une information concernant une relation entre au moins deux paramètres pris parmi le rythme de respiration, l'activité et le volume tidal,

12. Système selon la revendication 1, dans lequel l'indicateur résultant fournit une information concernant une relation entre une pression intravasculaire ou une saturation en oxygène du sang veineux et un niveau d'activité ou un ou plusieurs autre(s) paramètre(s) physiologique(s),

13. Système selon la revendication 1, comprenant :
un capteur d'activité implantable (555), qui est configuré de manière à détecter un signal d'activité physique à partir d'un sujet qui est soit un être humain, soit un animal, le signal d'activité physique tombant à l'intérieur d'une plage d'activité qui est divisée en classes de niveaux d'activité ;
un premier autre capteur physiologique implantable (505, 510, 515, 525, 560, 565), qui est configuré de manière à détecter de façon contemporaine un premier autre signal physiologique différent, à partir du sujet ;
un processeur (550), qui est configuré de manière à traiter le signal d'activité physique afin de définir une première période temporelle qui est associée à un premier niveau d'activité physique stationnaire du sujet, incluant l'identification, au sein de la première période temporelle, d'un premier temps de début auquel l'activité physique du sujet est considérée comme étant stationnaire au premier niveau d'activité physique stationnaire, et d'un premier temps de fin auquel le sujet quitte le premier niveau d'activité physique stationnaire, le premier niveau d'activité physique stationnaire correspondant à une classe de niveaux d'activité, le processeur (550) étant configuré de manière à traiter le signal d'activité physique afin de définir une deuxième période temporelle qui est associée à un deuxième niveau d'activité physique stationnaire du sujet, le deuxième niveau d'activité physique stationnaire du sujet correspondant à la même classe de niveaux d'activité que le premier niveau d'activité physique stationnaire du sujet, incluant l'identification, au sein de la deuxième période temporelle, d'un deuxième temps de début auquel l'activité physique du sujet est considérée comme étant stationnaire au deuxième niveau d'activité physique stationnaire, et d'un deuxième temps de fin auquel le sujet quitte le deuxième niveau d'activité physique stationnaire, et le processeur combinant des données qui proviennent du premier autre signal physiologique qui est également obtenu pendant les première et deuxième périodes temporelles, et le processeur déterminant un indicateur de résultat en utilisant les données combinées qui proviennent du premier autre signal physiologique.

14. Système selon la revendication 13, dans lequel le processeur (550) est configuré de manière à traiter le signal d'activité physique afin de définir une troisième période temporelle qui est associée à un troisième niveau d'activité physique stationnaire du sujet, incluant l'identification, au sein de la troisième période temporelle, d'un troisième temps de début auquel l'activité physique du sujet est considérée comme étant stationnaire au troisième niveau d'activité physique stationnaire, et d'un troisième temps de fin auquel le sujet quitte le troisième niveau d'activité physique stationnaire, le troisième niveau d'activité physique stationnaire correspondant à une classe de niveaux d'activité, et dans lequel le processeur est configuré de manière à traiter le signal d'activité physique afin de définir une quatrième période temporelle qui est associée à un quatrième niveau d'activité physique stationnaire du sujet, le quatrième niveau d'activité physique stationnaire du sujet correspondant à la même classe de niveaux d'activité que le premier niveau d'activité physique stationnaire du sujet, incluant l'identification, au sein de la quatrième période temporelle, d'un quatrième temps de début auquel l'activité physique du sujet est considérée comme étant stationnaire au quatrième niveau d'activité physique stationnaire, et d'un quatrième temps de fin auquel le sujet quitte le quatrième niveau d'activité physique stationnaire, et dans lequel le processeur est configuré de manière à combiner des données qui proviennent de l'autre signal physiologique qui est également obtenu pendant les troisième et quatrième périodes temporelles, et dans lequel le processeur détermine un deuxième indicateur résultant en utilisant les données combinées qui proviennent du premier autre signal physiologique qui est obtenu pendant les troisième et quatrième périodes temporelles, et dans lequel le processeur est configuré de manière à utiliser les premier et deuxième indicateurs résultants afin de surveiller une condition physiologique du sujet.

15. Système selon la revendication 13, dans lequel le processeur (550) est configuré de manière à combiner des données qui proviennent du premier autre signal physiologique qui est obtenu pendant les première et deuxième périodes temporelles en déterminant une première moyenne de données qui sont obtenues pendant la première période temporelle, en obtenant une deuxième moyenne de données qui sont obtenues pendant la deuxième période temporelle et en combinant la première moyenne et la deuxième moyenne.

16. Système selon la revendication 13, dans lequel le processeur (550) est configuré de manière à déterminer le premier indicateur résultant en utilisant les données combinées qui proviennent du premier autre signal physiologique en déterminant un rythme cardiaque, un volume systolique, un volume tidal ou un rythme de respiration pour un niveau d'activité stationnaire.

17. Système selon la revendication 13, dans lequel le processeur (550) est configuré de manière à déterminer le premier indicateur résultant en utilisant les données combinées qui proviennent du premier autre signal physiologique en déterminant une saturation en oxygène du sang veineux ou une pression diastolique d'artère pulmonaire pour un niveau d'activité stationnaire.

18. Système selon la revendication 1, dans lequel le processeur (550) est configuré de manière à déterminer si oui ou non le niveau d'activité candidat était stationnaire en déterminant si oui ou non le niveau d'activité candidat est à l'intérieur d'une première plage d'un niveau d'activité précédent, en déterminant si oui ou non le niveau d'activité candidat est à l'intérieur d'une seconde plage d'une séquence de niveaux d'activité précédents, la seconde plage étant plus grande que la première plage, et en déterminant si des niveaux d'activité présentent une tendance croissante ou décroissante en utilisant des niveaux d'activité détectés avant et après le niveau d'activité candidat.

19. Système selon la revendication 1, dans lequel le processeur est configuré de manière à déterminer si oui ou non le niveau d'activité candidat était stationnaire en déterminant si oui ou non la valeur de classe pour le niveau d'activité candidat est à l'intérieur d'une première plage d'une valeur de classe pour un niveau d'activité précédent, en déterminant si oui ou non la valeur de classe pour le niveau d'activité candidat est à l'intérieur d'une seconde plage de valeurs de classe pour une séquence de niveaux d'activité précédents, la seconde plage étant plus grande que la première plage, et en déterminant si des niveaux d'activité présentent une tendance croissante ou décroissante en utilisant des valeurs de classe qui proviennent de niveaux d'activité détectés avant et après le niveau d'activité candidat.

20. Système selon la revendication 19, dans lequel la première plage est une classe et la seconde plage est deux classes.

21. Système selon la revendication 1, comprenant en outre un indicateur résultant qui est déterminé en utilisant les moyennes de données physiologiques agrégées.

22. Système selon la revendication 21, dans lequel l'indicateur résultant est déterminé en utilisant les moyennes de données physiologiques agrégées afin d'agréger des données de rythme cardiaque, de volume systolique, de volume tidal, de rythme de respiration, de pression diastolique d'artère pulmonaire, ou un niveau d'oxygène du sang veineux.

23. Système selon la revendication 1, dans lequel le processeur (550) est configuré de manière à déterminer si oui ou non le niveau d'activité candidat était stationnaire en utilisant des valeurs de classe qui proviennent de niveaux d'activité détectés avant et après le niveau d'activité candidat en considérant le niveau d'activité candidat comme n'étant pas stationnaire si des niveaux d'activité qui proviennent d'une quantité de temps spécifiée qui précède le niveau d'activité candidat ne sont pas à l'intérieur d'une plage spécifiée.

24. Procédé assisté par machine comprenant :
la détection d'un signal d'activité physique, en utilisant un dispositif médical implantable (500), à partir d'un sujet qui est soit un être humain, soit un animal ;
la détection de manière simultanée d'un premier autre signal physiologique différent, à partir du sujet, en utilisant le dispositif médical implantable;
le traitement du signal d'activité physique afin de définir une première période temporelle qui est associée à un premier niveau d'activité physique stationnaire du sujet, incluant l'identification, au sein de la première période temporelle, d'un premier temps de début auquel l'activité physique du sujet est considérée comme étant stationnaire au premier niveau d'activité physique stationnaire, et d'un premier temps de fin auquel le sujet quitte le premier niveau d'activité physique stationnaire ;
l'obtention d'un premier indicateur en combinant des données qui proviennent du premier autre signal physiologique qui est obtenu pendant la première période temporelle ;
le traitement du signal d'activité physique afin de définir une deuxième période temporelle qui est associée à un deuxième niveau d'activité physique stationnaire du sujet, incluant l'identification, au sein de la deuxième période temporelle, d'un deuxième temps de début auquel l'activité physique du sujet est considérée comme étant stationnaire au deuxième niveau d'activité physique stationnaire, et d'un deuxième temps de fin auquel le sujet quitte le deuxième niveau d'activité physique stationnaire ;
l'obtention d'un deuxième indicateur en combinant des données qui proviennent du premier autre signal physiologique qui est obtenu pendant la deuxième période temporelle ; et
l'utilisation des premier et deuxième indicateurs afin de fournir un indicateur résultant,
le signal d'activité physique tombant à l'intérieur d'une plage d'activité qui est divisée selon des classes de niveaux d'activité, dans lequel :
le premier niveau d'activité physique stationnaire correspond à une classe de niveaux d'activité qui est associée à une plage de niveaux d'activité ;
le deuxième niveau d'activité physique stationnaire du sujet correspond à la même classe de niveaux d'activité que le premier niveau d'activité physique stationnaire du sujet ; et dans lequel :
les données qui proviennent du premier autre signal physiologique qui est également obtenu pendant les première et deuxième périodes temporelles sont combinées ; et
les données combinées qui proviennent du premier autre signal physiologique sont utilisées afin de fournir un indicateur résultant, dans lequel :
le procédé comprend également :
la détermination, pour un niveau d'activité candidat, d'une valeur de classe de niveaux d'activité qui correspond à une plage de valeurs de niveau d'activité ;
la détermination de si oui ou non le niveau d'activité candidat était stationnaire en utilisant des valeurs de classe qui proviennent de niveaux d'activité qui sont détectés avant et après le niveau d'activité candidat ;
si le niveau d'activité candidat était stationnaire, l'ajout, à un ou plusieurs registre(s) de sommes, de données physiologiques détectées de façon contemporaine par rapport au niveau d'activité candidat en utilisant un dispositif médical implantable, et l'incrémentation d'un ou de plusieurs compteur(s) ;
si le niveau d'activité candidat n'était pas stationnaire et si un niveau d'activité précédent était stationnaire ;
le calcul de la moyenne des données physiologiques en utilisant les un ou plusieurs registre(s) et les un ou plusieurs dompteur(5) ;
l'agrégation des données physiologiques moyennées avec des données physiologiques précédentes qui sont détectées à un niveau d'activité qui correspond à la même valeur de classe ; et
le stockage de moyennes de données physiologiques agrégées,

25. Procédé selon la revendication 24, dans lequel le deuxième niveau d'activité physique stationnaire correspond à une classe de niveaux d'activité différente de la classe de niveaux d'activité du premier niveau d'activité physique stationnaire.

26. Procédé selon la revendication 24, dans lequel le deuxième niveau d'activité physique stationnaire correspond à la même classe de niveaux d'activité que la classe de niveaux d'activité du premier niveau d'activité physique stationnaire.

27. Procédé selon la revendication 26, dans lequel la seconde période temporelle survient après la première période temporelle, et l'obtention d'un deuxième indicateur en combinant des données qui proviennent du premier autre signal physiologique qui est obtenu pendant la deuxième période temporelle inclut la combinaison de données qui proviennent du premier autre signal physiologique qui est obtenu pendant la deuxième période temporelle avec des données qui proviennent du premier autre signal physiologique qui est obtenu pendant la première période temporelle.

28. Procédé selon la revendication 27, comprenant en outre :
le traitement du signal d'activité physique afin de définir une troisième période temporelle qui est associée à un troisième niveau d'activité physique stationnaire du sujet, incluant l'identification, au sein de la troisième période temporelle, d'un troisième temps de début auquel l'activité physique du sujet est considérée comme étant stationnaire au troisième niveau d'activité physique stationnaire, et d'un troisième temps de fin auquel le sujet quitte le troisième niveau d'activité physique stationnaire, la troisième période temporelle survenant entre la première période temporelle et la deuxième période temporelle ; et
l'obtention d'un troisième indicateur en combinant des données qui proviennent du premier autre signal physiologique qui est obtenu prenant la troisième période temporelle.

29. Procédé selon la revendication 24, comprenant en outre la détection d'un second autre signal physiologique, à partir du sujet, en utilisant le dispositif médical implantable, et l'obtention de troisième et quatrième indicateurs en combinant des données qui proviennent du second autre signal physiologique qui est obtenu pendant les première et deuxième périodes temporelles respectives, dans lequel l'utilisation des premier et deuxième indicateurs afin de fournir un indicateur résultant inclut l'utilisation des premier, deuxième, troisième et quatrième indicateurs.

30. Procédé selon la revendication 24, dans lequel l'obtention d'un premier indicateur en combinant des données qui proviennent du premier autre signal physiologique qui est obtenu pendant la première période temporelle inclut le calcul de la moyenne des données qui proviennent du premier autre signal physiologique qui est obtenu pendant la première période temporelle.

31. Procédé selon la revendication 24, dans lequel l'indicateur résultant inclut un décalage, une différence, une dérivée, une interpolation ou une intégrale.

32. Procédé selon la revendication 24, dans lequel l'indicateur résultant fournit une information concernant une relation entre le rythme cardiaque et l'activité.

33. Procédé selon la revendication 24, dans lequel l'indicateur résultant fournit une information concernant une relation entre le volume systolique et l'activité ou le rythme cardiaque.

34. Procédé selon la revendication 24, dans lequel l'indicateur résultant fournit une information concernant une relation entre au moins deux paramètres pris parmi le rythme de respiration, l'activité et le volume tidal.

35. Procédé selon la revendication 24, dans lequel l'indicateur résultant fournit une information concernant une relation entre une pression intravasculaire ou une saturation en oxygène du sang veineux et un niveau d'activité ou un ou plusieurs autre(s) paramètre(s) physiologique(s).

36. Procédé selon la revendication 24, comprenant en outre :
le traitement du signal d'activité physique afin de définir une troisième période temporelle qui est associée à un troisième niveau d'activité physique stationnaire du sujet, incluant l'identification, au sein de la troisième période temporelle, d'un troisième temps de début auquel l'activité physique du sujet est considérée comme étant stationnaire au troisième niveau d'activité physique stationnaire, et d'un troisième temps de fin auquel le sujet quitte le troisième niveau d'activité physique stationnaire, le troisième niveau d'activité physique stationnaire correspondant à une classe de niveaux d'activité ;
le traitement du signal d'activité physique afin de définir une quatrième période temporelle qui est associée à un quatrième niveau d'activité physique stationnaire du sujet, le quatrième niveau d'activité physique stationnaire du sujet correspondant à la même classe de niveaux d'activité que le premier niveau d'activité physique stationnaire du sujet, incluant l'identification, au sein de la quatrième période temporelle, d'un quatrième temps de début auquel l'activité physique du sujet est considérée comme étant stationnaire au quatrième niveau d'activité physique stationnaire, et d'un quatrième temps de fin auquel le sujet quitte le quatrième niveau d'activité physique stationnaire ;
la combinaison de données qui proviennent du premier autre signal physiologique qui est également obtenu pendant les troisième et quatrième périodes temporelles ;
l'utilisation des données combinées qui proviennent du premier autre signal physiologique qui est obtenu pendant les troisième et quatrième périodes temporelles afin de fournir un deuxième indicateur résultant ;et
l'utilisation de l'indicateur résultant qui provient des première et deuxième périodes temporelles et de l'indicateur résultant qui provient des troisième et quatrième périodes temporelles afin de surveiller une condition physiologique du sujet.

37. Procédé selon la revendication 24, dans lequel la combinaison de données qui proviennent du premier autre signal physiologique qui est également obtenu pendant les première et deuxième périodes temporelles inclut la détermination d'une première moyenne de données qui sont obtenues pendant la première période temporelle, l'obtention d'une seconde moyenne de données qui sont obtenues pendant la deuxième période temporelle, et la combinaison de la première moyenne et de la seconde moyenne.

38. Procédé selon la revendication 24, dans lequel l'utilisation des données combinées qui proviennent du premier autre signal physiologique afin de fournir un indicateur résultant inclut la détermination d'un rythme cardiaque, d'un volume systolique, d'un volume tidal ou d'un rythme de respiration pour un niveau d'activité stationnaire.

39. Procédé selon la revendication 24, dans lequel l'utilisation des données combinées qui proviennent du premier autre signal physiologique afin de fournir un indicateur résultant inclut la détermination d'une saturation en oxygène du sang veineux ou d'une pression diastolique d'artère pulmonaire pour un niveau d'activité stationnaire.

40. Procédé selon la revendication 24, dans lequel la détermination de si oui ou non le niveau d'activité candidat était stationnaire inclut :
la détermination de si oui ou non le niveau d'activité candidat est à l'intérieur d'une première plage d'un niveau d'activité précédent ;
la détermination de si oui ou non le niveau d'activité candidat est à l'intérieur d'une seconde plage d'une séquence de niveaux d'activité précédents, la seconde plage étant plus grande que la première plage ; et
la détermination de si oui ou non des niveaux d'activité présentent une tendance croissante ou décroissante en utilisant des niveaux d'activité détectés avant et après le niveau d'activité candidat.

41. Procédé selon la revendication 24, dans lequel la détermination de si oui ou non le niveau d'activité candidat était stationnaire inclut :
la détermination de si oui ou non la valeur de classe pour le niveau d'activité candidat est à l'intérieur d'une première plage d'une valeur de classe pour un niveau d'activité précédent ;
la détermination de si oui ou non la valeur de classe pour le niveau d'activité candidat est à l'intérieur d'une seconde plage de valeurs de classe pour une séquence de niveaux d'activité précédents, la seconde plage étant plus grande que la première plage ; et
la détermination de si des niveaux d'activité présentent une tendance croissante ou décroissante en utilisant des valeurs de classe qui proviennent de niveaux d'activité détectés avant et après le niveau d'activité candidat.

42. Procédé selon la revendication 41, dans lequel la première plage est une classe et la seconde plage est deux classes.

43. Procédé selon la revendication 24, comprenant en outre l'utilisation des moyennes de données physiologiques agrégées afin de fournir un indicateur résultant.

44. Procédé selon la revendication 43, dans lequel l'utilisation des moyennes de données physiologiques agrégées afin de fournir un indicateur résultant inclut l'agrégation de données de rythme cardiaque, de volume systolique, de volume tidal, de rythme de respiration, de pression diastolique d'artère pulmonaire, ou du niveau d'oxygène du sang veineux.

45. Procédé selon la revendication 24, dans lequel la détermination de si oui ou non le niveau d'activité candidat était stationnaire en utilisant des valeurs de classe qui proviennent de niveaux d'activité qui sont détectés avant et après le niveau d'activité candidat inclut le fait de considérer le niveau d'activité candidat comme n'étant pas stationnaire si des niveaux d'activité qui proviennent d'une quantité de temps spécifiée qui précède le niveau d'activité candidat ne sont pas à l'intérieur d'une plage spécifiée.

46. Système selon la revendication 1, muni d'un second autre capteur de signal physiologique implantable, qui est configuré de manière à détecter un second autre signal physiologique à partir du sujet, et de manière à obtenir des troisième et quatrième indicateurs en utilisant des données qui proviennent du second autre signal physiologique qui est obtenu pendant les première et deuxième périodes temporelles respectives, dans lequel l'utilisation des premier et deuxième indicateurs afin de fournir l'indicateur résultant inclut l'utilisation des premier, deuxième, troisième et quatrième indicateurs afin de fournir l'indicateur résultant, dans lequel l'indicateur résultant représente un décalage croissant ou décroissant au niveau de la relation entre le premier autre signal physiologique et un second autre signal physiologique.

47. Système selon la revendication 1 ou 46, dans lequel le premier signal physiologique est indicatif d'au moins l'un des paramètres qui suivent : un rythme cardiaque, une sortie cardiaque, un volume systolique, un rythme de respiration, un volume tidal, une ventilation minute, une pression intravasculaire, ou une saturation en oxygène du sang veineux,

48. Système selon la revendication 46 ou 47, dans lequel le second signal physiologique est indicatif d'au moins l'un des paramètres qui suivent : un rythme cardiaque, une sortie cardiaque, un volume systolique, un rythme de respiration, un volume tidal, une ventilation minute, une pression intravasculaire, ou une saturation en oxygène du sang veineux.

49. Procédé selon la revendication 24, comprenant la détection d'un second autre signal physiologique, à partir du sujet, l'utilisation du dispositif médical implantable et l'obtention de troisième et quatrième indicateurs en combinant des données qui proviennent du second autre signal physiologique qui est obtenu pendant les première et deuxième périodes temporelles respectives, dans lequel l'utilisateur des premier et deuxième indicateurs afin de fournir un indicateur résultant inclut l'utilisation des premier, deuxième, troisième et quatrième indicateurs, dans lequel l'indicateur résultant représente un décalage croissant ou décroissant au niveau de la relation entre le premier autre signal physiologique et un second autre signal physiologique.

50. Procédé selon la revendication 24 ou 49, dans lequel le premier signal physiologique est indicatif d'au moins l'un des paramètres qui suivent : un rythme cardiaque, une sortie cardiaque, un volume systolique, un rythme de respiration, un volume tidal, une ventilation minute, une pression intravasculaire, ou une saturation en oxygène du sang veineux.

51. Procédé selon la revendication 49 ou 50, dans lequel le second signal physiologique est indicatif d'au moins l'un des paramètres qui suivent : un rythme cardiaque, une sortie cardiaque, un volume systolique, un rythme de respiration, un volume tidal, une ventilation minute, une pression intravasculaire, ou une saturation en oxygène du sang veineux.
